# EUROPEAN PATENT SPECIFICATION

(11) **EP 3 242 891 B1**
(45) Date of publication and mention of the grant of the patent: **18.08.2021**
(21) Application number: 16700287.2
(22) Date of filing: 04.01.2016
(51) Int. Cl.: C07K 16/28, C07K 16/46

(54) **MONOMERIC FC DOMAINS**
MONOMERE FC-DOMÄNEN
DOMAINES FC MONOMÈRES

(30) Priority: 05.01.2015 US 201562099634 P
(43) Date of publication of application: 15.11.2017
(73) Proprietor: Innate Pharma, 13009 Marseille (FR)
(72) Inventor: GAUTHIER, Laurent, 13008 Marseille (FR)
(74) Representative: Gallois, Valérie
(86) International application number: PCT/EP2016/050032
(87) International publication number: WO 2016/110468

(56) References cited:
- WO-A1-2008/012543
- WO-A1-2008/131242
- WO-A1-2010/045261
- WO-A1-2015/197593
- DALL'ACQUA WILLIAM ET AL: "Contribution of domain interface residues to the stability of antibody CH3 domain homodimers", BIOCHEMISTRY, AMERICAN CHEMICAL SOCIETY, US, vol. 37, no. 26, 30 June 1998 (1998-06-30) , pages 9266-9273, XP002245748, ISSN: 0006-2960, DOI: 10.1021/BI980270I

### SEQUENCE LISTING

<110> INNATE PHARMA
<120> MONOMERIC FC DOMAINS
<130> NTECH2
<150> US 62/099,634
   <151> 2015-01-05
<160> 29
<170> PatentIn version 3.5
<210> 1
   <211> 106
   <212> PRT
   <213> homo sapiens
<400> 1
   Gly Gln Pro Arg Glu Pro Gln Val Tyr Thr Leu Pro Pro Ser Arg Glu
   1               5                   10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly
               100                 105
<210> 2
   <211> 227
   <212> PRT
   <213> homo sapiens
<400> 2
   Gly Gln Pro Arg Glu Pro Gln Val Tyr Thr Leu Pro Pro Ser Arg Glu
   1               5                   10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Gly Gly Gly Gly Ser Gly
               100                 105                 110
   Gly Gly Gly Ser Gly Gly Gly Gly Ser Gly Gln Pro Arg Glu Pro Gln
           115                 120                 125
   Val Tyr Thr Leu Pro Pro Ser Arg Glu Glu Met Thr Lys Asn Gln Val
       130                 135                 140
   Ser Leu Thr Cys Leu Val Lys Gly Phe Tyr Pro Ser Asp Ile Ala Val
   145                 150                 155                 160
   Glu Trp Glu Ser Asn Gly Gln Pro Glu Asn Asn Tyr Lys Thr Thr Pro
                   165                 170                 175
   Pro Val Leu Asp Ser Asp Gly Ser Phe Phe Leu Tyr Ser Lys Leu Thr
               180                 185                 190
   Val Asp Lys Ser Arg Trp Gln Gln Gly Asn Val Phe Ser Cys Ser Val
           195                 200                 205
   Met His Glu Ala Leu His Asn His Tyr Thr Gln Lys Ser Leu Ser Leu
       210                 215                 220
   Ser Pro Gly
   225
<210> 3
   <211> 333
   <212> DNA
   <213> mus musculus
<400> 3
   gacattcagc tgacccaatc tccagcttct ttggctgtgt ctctagggca gagggccacc      60
   atctcctgca aggccagcca aagtgttgat tatgatggtg atagttattt gaactggtac     120
   caacagatac caggacagcc acccaaactc ctcatctatg atgcatccaa tctagtatct     180
   gggattccac ccaggtttag tggcagtggg tctgggacag acttcaccct caacatccat     240
   cctgtggaga aggtggatgc tgcaacctat cactgtcagc aaagtactga ggacccttgg     300
   acgttcggtg gaggcaccaa gctggaaatc aaa                                  333
<210> 4
   <211> 111
   <212> PRT
   <213> mus musculus
<400> 4
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys
               100                 105                 110
<210> 5
   <211> 372
   <212> DNA
   <213> mus musculus
<400> 5
   caggttcagc tgcagcagtc tggggctgag ctggtgcggc ctgggtcctc agtgaagatt      60
   tcctgcaaag catctggcta cgcattcagt agctactgga tgaactgggt gaagcagagg     120
   cctggacagg gtcttgagtg gattggacag atttggcctg gagatggtga tactaactac     180
   aacggaaagt tcaagggcaa ggccacactg actgcagacg aatcctccag cacagcctac     240
   atgcagctca gcagcctggc ctctgaggac tctgcggtct atttctgtgc aagacgagaa     300
   acgaccactg tcgggcgtta ttactatgct atggactact ggggtcaagg aaccacagtc     360
   accgtctcct ca                                                         372
<210> 6
   <211> 124
   <212> PRT
   <213> mus musculus
<400> 6
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Thr Val Thr Val Ser Ser
           115                 120
<210> 7
   <211> 116
   <212> PRT
   <213> mus musculus
<400> 7
   Glu Val Gln Leu Gln Gln Ser Gly Pro Glu Leu Val Lys Pro Gly Ala
   1 5 10 15
   Ser Val Lys Ile Ser Cys Lys Thr Ser Gly Tyr Thr Phe Thr Glu Tyr
   20 25 30
   Thr Met His Trp Val Lys Gln Ser His Gly Lys Ser Leu Glu Trp Ile
   35 40 45
   Gly Gly Ile Ser Pro Asn Ile Gly Gly Thr Ser Tyr Asn Gln Lys Phe
   50 55 60
   Lys Gly Lys Ala Thr Leu Thr Val Asp Lys Ser Ser Ser Thr Ala Tyr
   65 70 75 80
   Met Glu Leu Arg Ser Leu Thr Ser Glu Asp Ser Ala Val Tyr Tyr Cys
   85 90 95
   Ala Arg Arg Gly Gly Ser Phe Asp Tyr Trp Gly Gln Gly Thr Thr Leu
   100 105 110
   Thr Val Ser Ser
   115
<210> 8
   <211> 107
   <212> PRT
   <213> mus musculus
<400> 8
   Asp Ile Val Met Thr Gln Ser Pro Ala Thr Leu Ser Val Thr Pro Gly
   1               5                   10                  15
   Asp Arg Val Ser Leu Ser Cys Arg Ala Ser Gln Ser Ile Ser Asp Tyr
               20                  25                  30
   Leu His Trp Tyr Gln Gln Lys Ser His Glu Ser Pro Arg Leu Leu Ile
           35                  40                  45
   Lys Tyr Ala Ser Gln Ser Ile Ser Gly Ile Pro Ser Arg Phe Ser Gly
       50                  55                  60
   Ser Gly Ser Gly Ser Asp Phe Thr Leu Ser Ile Asn Ser Val Glu Pro
   65                  70                  75                  80
   Glu Asp Val Gly Val Tyr Tyr Cys Gln Asn Gly His Ser Phe Pro Leu
                   85                  90                  95
   Thr Phe Gly Ala Gly Thr Lys Leu Glu Leu Lys
               100                 105
<210> 9
   <211> 245
   <212> PRT
   <213> mus musculus
<400> 9
   Ser Thr Gly Ser Glu Val Gln Leu Gln Gln Ser Gly Pro Glu Leu Val
   1               5                   10                  15
   Lys Pro Gly Ala Ser Val Lys Ile Ser Cys Lys Thr Ser Gly Tyr Thr
               20                  25                  30
   Phe Thr Glu Tyr Thr Met His Trp Val Lys Gln Ser His Gly Lys Ser
           35                  40                  45
   Leu Glu Trp Ile Gly Gly Ile Ser Pro Asn Ile Gly Gly Thr Ser Tyr
       50                  55                  60
   Asn Gln Lys Phe Lys Gly Lys Ala Thr Leu Thr Val Asp Lys Ser Ser
   65                  70                  75                  80
   Ser Thr Ala Tyr Met Glu Leu Arg Ser Leu Thr Ser Glu Asp Ser Ala
                   85                  90                  95
   Val Tyr Tyr Cys Ala Arg Arg Gly Gly Ser Phe Asp Tyr Trp Gly Gln
               100                 105                 110
   Gly Thr Thr Leu Thr Val Ser Ser Val Glu Gly Gly Ser Gly Gly Ser
           115                 120                 125
   Gly Gly Ser Gly Gly Ser Gly Gly Val Asp Asp Ile Val Met Thr Gln
       130                 135                 140
   Ser Pro Ala Thr Leu Ser Val Thr Pro Gly Asp Arg Val Ser Leu Ser
   145                 150                 155                 160
   Cys Arg Ala Ser Gln Ser Ile Ser Asp Tyr Leu His Trp Tyr Gln Gln
                   165                 170                 175
   Lys Ser His Glu Ser Pro Arg Leu Leu Ile Lys Tyr Ala Ser Gln Ser
               180                 185                 190
   Ile Ser Gly Ile Pro Ser Arg Phe Ser Gly Ser Gly Ser Gly Ser Asp
           195                 200                 205
   Phe Thr Leu Ser Ile Asn Ser Val Glu Pro Glu Asp Val Gly Val Tyr
       210                 215                 220
   Tyr Cys Gln Asn Gly His Ser Phe Pro Leu Thr Phe Gly Ala Gly Thr
   225                 230                 235                 240
   Lys Leu Glu Leu Lys
                   245
<210> 10
   <211> 837
   <212> PRT
   <213> Artificial
<220>
   <223> Chimeric mus musculus homo sapiens
<400> 10
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ser Gln Val
           115                 120                 125
   Gln Leu Gln Gln Ser Gly Ala Glu Leu Val Arg Pro Gly Ser Ser Val
       130                 135                 140
   Lys Ile Ser Cys Lys Ala Ser Gly Tyr Ala Phe Ser Ser Tyr Trp Met
   145                 150                 155                 160
   Asn Trp Val Lys Gln Arg Pro Gly Gln Gly Leu Glu Trp Ile Gly Gln
                   165                 170                 175
   Ile Trp Pro Gly Asp Gly Asp Thr Asn Tyr Asn Gly Lys Phe Lys Gly
               180                 185                 190
   Lys Ala Thr Leu Thr Ala Asp Glu Ser Ser Ser Thr Ala Tyr Met Gln
           195                 200                 205
   Leu Ser Ser Leu Ala Ser Glu Asp Ser Ala Val Tyr Phe Cys Ala Arg
       210                 215                 220
   Arg Glu Thr Thr Thr Val Gly Arg Tyr Tyr Tyr Ala Met Asp Tyr Trp
   225                 230                 235                 240
   Gly Gln Gly Thr Thr Val Thr Val Ser Ser Gly Gly Gly Ser Ser Ala
                   245                 250                 255
   Pro Glu Leu Leu Gly Gly Pro Ser Val Phe Leu Phe Pro Pro Lys Pro
               260                 265                 270
   Lys Asp Thr Leu Met Ile Ser Arg Thr Pro Glu Val Thr Cys Val Val
           275                 280                 285
   Val Asp Val Ser His Glu Asp Pro Glu Val Lys Phe Asn Trp Tyr Val
       290                 295                 300
   Asp Gly Val Glu Val His Asn Ala Lys Thr Lys Pro Arg Glu Glu Gln
   305                 310                 315                 320
   Tyr Asn Ser Thr Tyr Arg Val Val Ser Val Leu Thr Val Leu His Gln
                   325                 330                 335
   Asp Trp Leu Asn Gly Lys Glu Tyr Lys Cys Lys Val Ser Asn Lys Ala
               340                 345                 350
   Leu Pro Ala Pro Ile Glu Lys Thr Ile Ser Lys Ala Lys Gly Gln Pro
           355                 360                 365
   Arg Glu Pro Gln Val Tyr Thr Leu Pro Pro Ser Arg Glu Glu Met Thr
       370                 375                 380
   Lys Asn Gln Val Ser Leu Thr Cys Leu Val Lys Gly Phe Tyr Pro Ser
   385                 390                 395                 400
   Asp Ile Ala Val Glu Trp Glu Ser Asn Gly Gln Pro Glu Asn Asn Tyr
                   405                 410                 415
   Lys Thr Thr Pro Pro Val Leu Asp Ser Asp Gly Ser Phe Phe Leu Tyr
               420                 425                 430
   Ser Lys Leu Thr Val Asp Lys Ser Arg Trp Gln Gln Gly Asn Val Phe
           435                 440                 445
   Ser Cys Ser Val Met His Glu Ala Leu His Asn His Tyr Thr Gln Lys
       450                 455                 460
   Ser Leu Ser Leu Ser Pro Gly Gly Gly Gly Gly Ser Gly Gly Gly Gly
   465                 470                 475                 480
   Ser Gly Gly Gly Gly Ser Gly Gln Pro Arg Glu Pro Gln Val Tyr Thr
   485 490 495
   Leu Pro Pro Ser Arg Glu Glu Met Thr Lys Asn Gln Val Ser Leu Thr
   500 505 510
   Cys Leu Val Lys Gly Phe Tyr Pro Ser Asp Ile Ala Val Glu Trp Glu
   515 520 525
   Ser Asn Gly Gln Pro Glu Asn Asn Tyr Lys Thr Thr Pro Pro Val Leu
   530 535 540
   Asp Ser Asp Gly Ser Phe Phe Leu Tyr Ser Lys Leu Thr Val Asp Lys
   545 550 555 560
   Ser Arg Trp Gln Gln Gly Asn Val Phe Ser Cys Ser Val Met His Glu
   565 570 575
   Ala Leu His Asn His Tyr Thr Gln Lys Ser Leu Ser Leu Ser Pro Gly
   580 585 590
   Ser Thr Gly Ser Glu Val Gln Leu Gln Gln Ser Gly Pro Glu Leu Val
   595 600 605
   Lys Pro Gly Ala Ser Val Lys Ile Ser Cys Lys Thr Ser Gly Tyr Thr
   610 615 620
   Phe Thr Glu Tyr Thr Met His Trp Val Lys Gln Ser His Gly Lys Ser
   625 630 635 640
   Leu Glu Trp Ile Gly Gly Ile Ser Pro Asn Ile Gly Gly Thr Ser Tyr
   645 650 655
   Asn Gln Lys Phe Lys Gly Lys Ala Thr Leu Thr Val Asp Lys Ser Ser
   660 665 670
   Ser Thr Ala Tyr Met Glu Leu Arg Ser Leu Thr Ser Glu Asp Ser Ala
   675 680 685
   Val Tyr Tyr Cys Ala Arg Arg Gly Gly Ser Phe Asp Tyr Trp Gly Gln
   690 695 700
   Gly Thr Thr Leu Thr Val Ser Ser Val Glu Gly Gly Ser Gly Gly Ser
   705 710 715 720
   Gly Gly Ser Gly Gly Ser Gly Gly Val Asp Asp Ile Val Met Thr Gln
                   725                 730                 735
   Ser Pro Ala Thr Leu Ser Val Thr Pro Gly Asp Arg Val Ser Leu Ser
               740                 745                 750
   Cys Arg Ala Ser Gln Ser Ile Ser Asp Tyr Leu His Trp Tyr Gln Gln
           755                 760                 765
   Lys Ser His Glu Ser Pro Arg Leu Leu Ile Lys Tyr Ala Ser Gln Ser
       770                 775                 780
   Ile Ser Gly Ile Pro Ser Arg Phe Ser Gly Ser Gly Ser Gly Ser Asp
   785                 790                 795                 800
   Phe Thr Leu Ser Ile Asn Ser Val Glu Pro Glu Asp Val Gly Val Tyr
                   805                 810                 815
   Tyr Cys Gln Asn Gly His Ser Phe Pro Leu Thr Phe Gly Ala Gly Thr
               820                 825                 830
   Lys Leu Glu Leu Lys
           835
<210> 11
   <211> 837
   <212> PRT
   <213> artificial
<220>
   <223> Chimeric mus musculus homo sapiens
<400> 11
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ser Gln Val
           115                 120                 125
   Gln Leu Gln Gln Ser Gly Ala Glu Leu Val Arg Pro Gly Ser Ser Val
       130                 135                 140
   Lys Ile Ser Cys Lys Ala Ser Gly Tyr Ala Phe Ser Ser Tyr Trp Met
   145                 150                 155                 160
   Asn Trp Val Lys Gln Arg Pro Gly Gln Gly Leu Glu Trp Ile Gly Gln
                   165                 170                 175
   Ile Trp Pro Gly Asp Gly Asp Thr Asn Tyr Asn Gly Lys Phe Lys Gly
               180                 185                 190
   Lys Ala Thr Leu Thr Ala Asp Glu Ser Ser Ser Thr Ala Tyr Met Gln
           195                 200                 205
   Leu Ser Ser Leu Ala Ser Glu Asp Ser Ala Val Tyr Phe Cys Ala Arg
       210                 215                 220
   Arg Glu Thr Thr Thr Val Gly Arg Tyr Tyr Tyr Ala Met Asp Tyr Trp
   225                 230                 235                 240
   Gly Gln Gly Thr Thr Val Thr Val Ser Ser Gly Gly Gly Ser Ser Ala
                   245                 250                 255
   Pro Glu Leu Leu Gly Gly Pro Ser Val Phe Leu Phe Pro Pro Lys Pro
               260                 265                 270
   Lys Asp Thr Leu Met Ile Ser Arg Thr Pro Glu Val Thr Cys Val Val
           275                 280                 285
   Val Asp Val Ser His Glu Asp Pro Glu Val Lys Phe Asn Trp Tyr Val
       290                 295                 300
   Asp Gly Val Glu Val His Asn Ala Lys Thr Lys Pro Arg Glu Glu Gln
   305                 310                 315                 320
   Tyr Ser Ser Thr Tyr Arg Val Val Ser Val Leu Thr Val Leu His Gln
                   325                 330                 335
   Asp Trp Leu Asn Gly Lys Glu Tyr Lys Cys Lys Val Ser Asn Lys Ala
               340                 345                 350
   Leu Pro Ala Pro Ile Glu Lys Thr Ile Ser Lys Ala Lys Gly Gln Pro
           355                 360                 365
   Arg Glu Pro Gln Val Tyr Thr Leu Pro Pro Ser Arg Glu Glu Met Thr
       370                 375                 380
   Lys Asn Gln Val Ser Leu Thr Cys Leu Val Lys Gly Phe Tyr Pro Ser
   385                 390                 395                 400
   Asp Ile Ala Val Glu Trp Glu Ser Asn Gly Gln Pro Glu Asn Asn Tyr
                   405                 410                 415
   Lys Thr Thr Pro Pro Val Leu Asp Ser Asp Gly Ser Phe Phe Leu Tyr
               420                 425                 430
   Ser Lys Leu Thr Val Asp Lys Ser Arg Trp Gln Gln Gly Asn Val Phe
           435                 440                 445
   Ser Cys Ser Val Met His Glu Ala Leu His Asn His Tyr Thr Gln Lys
       450                 455                 460
   Ser Leu Ser Leu Ser Pro Gly Gly Gly Gly Gly Ser Gly Gly Gly Gly
   465                 470                 475                 480
   Ser Gly Gly Gly Gly Ser Gly Gln Pro Arg Glu Pro Gln Val Tyr Thr
                   485                 490                 495
   Leu Pro Pro Ser Arg Glu Glu Met Thr Lys Asn Gln Val Ser Leu Thr
               500                 505                 510
   Cys Leu Val Lys Gly Phe Tyr Pro Ser Asp Ile Ala Val Glu Trp Glu
           515                 520                 525
   Ser Asn Gly Gln Pro Glu Asn Asn Tyr Lys Thr Thr Pro Pro Val Leu
       530                 535                 540
   Asp Ser Asp Gly Ser Phe Phe Leu Tyr Ser Lys Leu Thr Val Asp Lys
   545                 550                 555                 560
   Ser Arg Trp Gln Gln Gly Asn Val Phe Ser Cys Ser Val Met His Glu
                   565                 570                 575
   Ala Leu His Asn His Tyr Thr Gln Lys Ser Leu Ser Leu Ser Pro Gly
               580                 585                 590
   Ser Thr Gly Ser Glu Val Gln Leu Gln Gln Ser Gly Pro Glu Leu Val
           595                 600                 605
   Lys Pro Gly Ala Ser Val Lys Ile Ser Cys Lys Thr Ser Gly Tyr Thr
       610                 615                 620
   Phe Thr Glu Tyr Thr Met His Trp Val Lys Gln Ser His Gly Lys Ser
   625                 630                 635                 640
   Leu Glu Trp Ile Gly Gly Ile Ser Pro Asn Ile Gly Gly Thr Ser Tyr
                   645                 650                 655
   Asn Gln Lys Phe Lys Gly Lys Ala Thr Leu Thr Val Asp Lys Ser Ser
               660                 665                 670
   Ser Thr Ala Tyr Met Glu Leu Arg Ser Leu Thr Ser Glu Asp Ser Ala
           675                 680                 685
   Val Tyr Tyr Cys Ala Arg Arg Gly Gly Ser Phe Asp Tyr Trp Gly Gln
       690                 695                 700
   Gly Thr Thr Leu Thr Val Ser Ser Val Glu Gly Gly Ser Gly Gly Ser
   705                 710                 715                 720
   Gly Gly Ser Gly Gly Ser Gly Gly Val Asp Asp Ile Val Met Thr Gln
                   725                 730                 735
   Ser Pro Ala Thr Leu Ser Val Thr Pro Gly Asp Arg Val Ser Leu Ser
               740                 745                 750
   Cys Arg Ala Ser Gln Ser Ile Ser Asp Tyr Leu His Trp Tyr Gln Gln
           755                 760                 765
   Lys Ser His Glu Ser Pro Arg Leu Leu Ile Lys Tyr Ala Ser Gln Ser
       770                 775                 780
   Ile Ser Gly Ile Pro Ser Arg Phe Ser Gly Ser Gly Ser Gly Ser Asp
   785                 790                 795                 800
   Phe Thr Leu Ser Ile Asn Ser Val Glu Pro Glu Asp Val Gly Val Tyr
                   805                 810                 815
   Tyr Cys Gln Asn Gly His Ser Phe Pro Leu Thr Phe Gly Ala Gly Thr
               820                 825                 830
   Lys Leu Glu Leu Lys
           835
<210> 12
   <211> 218
   <212> PRT
   <213> artificial
<220>
   <223> Chimeric mus musculus homo sapiens
<400> 12
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
   210 215
<210> 13
   <211> 801
   <212> PRT
   <213> Artificial
<220>
   <223> Chimeric mus musculus homo sapiens
<400> 13
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Thr Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Arg Val Glu Pro
       210                 215                 220
   Lys Ser Cys Asp Lys Thr His Thr Cys Pro Pro Cys Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Ser
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
                   405                 410                 415
   Leu Thr Val Asp Lys Ser Arg Trp Gln Gln Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Pro Gly Gly Gly Gly Gly Ser Gly Gly Gly Gly Ser Gly
       450                 455                 460
   Gly Gly Gly Ser Gly Gln Pro Arg Glu Pro Gln Val Tyr Thr Leu Pro
   465                 470                 475                 480
   Pro Ser Arg Glu Glu Met Thr Lys Asn Gln Val Ser Leu Thr Cys Leu
                   485                 490                 495
   Val Lys Gly Phe Tyr Pro Ser Asp Ile Ala Val Glu Trp Glu Ser Asn
               500                 505                 510
   Gly Gln Pro Glu Asn Asn Tyr Lys Thr Thr Pro Pro Val Leu Asp Ser
           515                 520                 525
   Asp Gly Ser Phe Phe Leu Tyr Ser Lys Leu Thr Val Asp Lys Ser Arg
       530                 535                 540
   Trp Gln Gln Gly Asn Val Phe Ser Cys Ser Val Met His Glu Ala Leu
   545                 550                 555                 560
   His Asn His Tyr Thr Gln Lys Ser Leu Ser Leu Ser Pro Gly Ser Thr
                   565                 570                 575
   Gly Ser Glu Val Gln Leu Gln Gln Ser Gly Pro Glu Leu Val Lys Pro
               580                 585                 590
   Gly Ala Ser Val Lys Ile Ser Cys Lys Thr Ser Gly Tyr Thr Phe Thr
           595                 600                 605
   Glu Tyr Thr Met His Trp Val Lys Gln Ser His Gly Lys Ser Leu Glu
       610                 615                 620
   Trp Ile Gly Gly Ile Ser Pro Asn Ile Gly Gly Thr Ser Tyr Asn Gln
   625                 630                 635                 640
   Lys Phe Lys Gly Lys Ala Thr Leu Thr Val Asp Lys Ser Ser Ser Thr
                   645                 650                 655
   Ala Tyr Met Glu Leu Arg Ser Leu Thr Ser Glu Asp Ser Ala Val Tyr
               660                 665                 670
   Tyr Cys Ala Arg Arg Gly Gly Ser Phe Asp Tyr Trp Gly Gln Gly Thr
           675                 680                 685
   Thr Leu Thr Val Ser Ser Arg Thr Val Ala Ala Pro Ser Val Phe Ile
       690                 695                 700
   Phe Pro Pro Ser Asp Glu Gln Leu Lys Ser Gly Thr Ala Ser Val Val
   705 710 715 720
   Cys Leu Leu Asn Asn Phe Tyr Pro Arg Glu Ala Lys Val Gln Trp Lys
   725 730 735
   Val Asp Asn Ala Leu Gln Ser Gly Asn Ser Gln Glu Ser Val Thr Glu
   740 745 750
   Gln Asp Ser Lys Asp Ser Thr Tyr Ser Leu Ser Ser Thr Leu Thr Leu
   755 760 765
   Ser Lys Ala Asp Tyr Glu Lys His Lys Val Tyr Ala Cys Glu Val Thr
   770 775 780
   His Gln Gly Leu Ser Ser Pro Val Thr Lys Ser Phe Asn Arg Gly Glu
   785 790 795 800
   Cys
<210> 14
   <211> 214
   <212> PRT
   <213> artificial
<220>
   <223> chimeric mus musculus homo sapiens
<400> 14
   Asp Ile Val Met Thr Gln Ser Pro Ala Thr Leu Ser Val Thr Pro Gly
   1 5 10 15
   Asp Arg Val Ser Leu Ser Cys Arg Ala Ser Gln Ser Ile Ser Asp Tyr
   20 25 30
   Leu His Trp Tyr Gln Gln Lys Ser His Glu Ser Pro Arg Leu Leu Ile
   35 40 45
   Lys Tyr Ala Ser Gln Ser Ile Ser Gly Ile Pro Ser Arg Phe Ser Gly
   50 55 60
   Ser Gly Ser Gly Ser Asp Phe Thr Leu Ser Ile Asn Ser Val Glu Pro
   65 70 75 80
   Glu Asp Val Gly Val Tyr Tyr Cys Gln Asn Gly His Ser Phe Pro Leu
   85 90 95
   Thr Phe Gly Ala Gly Thr Lys Leu Glu Leu Lys Ala Ser Thr Lys Gly
               100                 105                 110
   Pro Ser Val Phe Pro Leu Ala Pro Ser Ser Lys Ser Thr Ser Gly Gly
           115                 120                 125
   Thr Ala Ala Leu Gly Cys Leu Val Lys Asp Tyr Phe Pro Glu Pro Val
       130                 135                 140
   Thr Val Ser Trp Asn Ser Gly Ala Leu Thr Ser Gly Val His Thr Phe
   145                 150                 155                 160
   Pro Ala Val Leu Gln Ser Ser Gly Leu Tyr Ser Leu Ser Ser Val Val
                   165                 170                 175
   Thr Val Pro Ser Ser Ser Leu Gly Thr Gln Thr Tyr Ile Cys Asn Val
               180                 185                 190
   Asn His Lys Pro Ser Asn Thr Lys Val Asp Lys Arg Val Glu Pro Lys
           195                 200                 205
   Ser Cys Asp Lys Thr His
       210
<210> 15
   <211> 801
   <212> PRT
   <213> artificial
<220>
   <223> Chimeric mus musculus homo sapiens
<400> 15
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
   85 90 95
   Ala Arg Arg Glu Thr Thr Thr Val Gly Arg Tyr Tyr Tyr Ala Met Asp
   100 105 110
   Tyr Trp Gly Gln Gly Thr Thr Val Thr Val Ser Ser Ala Ser Thr Lys
   115 120 125
   Gly Pro Ser Val Phe Pro Leu Ala Pro Ser Ser Lys Ser Thr Ser Gly
   130 135 140
   Gly Thr Ala Ala Leu Gly Cys Leu Val Lys Asp Tyr Phe Pro Glu Pro
   145 150 155 160
   Val Thr Val Ser Trp Asn Ser Gly Ala Leu Thr Ser Gly Val His Thr
   165 170 175
   Phe Pro Ala Val Leu Gln Ser Ser Gly Leu Tyr Ser Leu Ser Ser Val
   180 185 190
   Val Thr Val Pro Ser Ser Ser Leu Gly Thr Gln Thr Tyr Ile Cys Asn
   195 200 205
   Val Asn His Lys Pro Ser Asn Thr Lys Val Asp Lys Arg Val Glu Pro
   210 215 220
   Lys Ser Cys Asp Lys Thr His Thr Ser Pro Pro Ser Pro Ala Pro Glu
   225 230 235 240
   Leu Leu Gly Gly Pro Ser Val Phe Leu Phe Pro Pro Lys Pro Lys Asp
   245 250 255
   Thr Leu Met Ile Ser Arg Thr Pro Glu Val Thr Cys Val Val Val Asp
   260 265 270
   Val Ser His Glu Asp Pro Glu Val Lys Phe Asn Trp Tyr Val Asp Gly
   275 280 285
   Val Glu Val His Asn Ala Lys Thr Lys Pro Arg Glu Glu Gln Tyr Ser
   290 295 300
   Ser Thr Tyr Arg Val Val Ser Val Leu Thr Val Leu His Gln Asp Trp
   305 310 315 320
   Leu Asn Gly Lys Glu Tyr Lys Cys Lys Val Ser Asn Lys Ala Leu Pro
   325 330 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
                   405                 410                 415
   Leu Thr Val Asp Lys Ser Arg Trp Gln Gln Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Pro Gly Gly Gly Gly Gly Ser Gly Gly Gly Gly Ser Gly
       450                 455                 460
   Gly Gly Gly Ser Gly Gln Pro Arg Glu Pro Gln Val Tyr Thr Leu Pro
   465                 470                 475                 480
   Pro Ser Arg Glu Glu Met Thr Lys Asn Gln Val Ser Leu Thr Cys Leu
                   485                 490                 495
   Val Lys Gly Phe Tyr Pro Ser Asp Ile Ala Val Glu Trp Glu Ser Asn
               500                 505                 510
   Gly Gln Pro Glu Asn Asn Tyr Lys Thr Thr Pro Pro Val Leu Asp Ser
           515                 520                 525
   Asp Gly Ser Phe Phe Leu Tyr Ser Lys Leu Thr Val Asp Lys Ser Arg
       530                 535                 540
   Trp Gln Gln Gly Asn Val Phe Ser Cys Ser Val Met His Glu Ala Leu
   545                 550                 555                 560
   His Asn His Tyr Thr Gln Lys Ser Leu Ser Leu Ser Pro Gly Ser Thr
                   565                 570                 575
   Gly Ser Glu Val Gln Leu Gln Gln Ser Gly Pro Glu Leu Val Lys Pro
               580                 585                 590
   Gly Ala Ser Val Lys Ile Ser Cys Lys Thr Ser Gly Tyr Thr Phe Thr
   595 600 605
   Glu Tyr Thr Met His Trp Val Lys Gln Ser His Gly Lys Ser Leu Glu
   610 615 620
   Trp Ile Gly Gly Ile Ser Pro Asn Ile Gly Gly Thr Ser Tyr Asn Gln
   625 630 635 640
   Lys Phe Lys Gly Lys Ala Thr Leu Thr Val Asp Lys Ser Ser Ser Thr
   645 650 655
   Ala Tyr Met Glu Leu Arg Ser Leu Thr Ser Glu Asp Ser Ala Val Tyr
   660 665 670
   Tyr Cys Ala Arg Arg Gly Gly Ser Phe Asp Tyr Trp Gly Gln Gly Thr
   675 680 685
   Thr Leu Thr Val Ser Ser Arg Thr Val Ala Ala Pro Ser Val Phe Ile
   690 695 700
   Phe Pro Pro Ser Asp Glu Gln Leu Lys Ser Gly Thr Ala Ser Val Val
   705 710 715 720
   Cys Leu Leu Asn Asn Phe Tyr Pro Arg Glu Ala Lys Val Gln Trp Lys
   725 730 735
   Val Asp Asn Ala Leu Gln Ser Gly Asn Ser Gln Glu Ser Val Thr Glu
   740 745 750
   Gln Asp Ser Lys Asp Ser Thr Tyr Ser Leu Ser Ser Thr Leu Thr Leu
   755 760 765
   Ser Lys Ala Asp Tyr Glu Lys His Lys Val Tyr Ala Cys Glu Val Thr
   770 775 780
   His Gln Gly Leu Ser Ser Pro Val Thr Lys Ser Phe Asn Arg Gly Glu
   785 790 795 800
   Cys
<210> 16
   <211> 796
   <212> PRT
   <213> artificial
<220>
   <223> Chimeric mus musculus homo sapiens
<400> 16
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Thr Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Arg Val Glu Pro
       210                 215                 220
   Lys Ser Cys Gly Gly Gly Ser Ser Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Ser Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Glu Glu Met Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Gly Gly Gly Gly Ser Gly Gly Gly Gly Ser Gly Gly Gly Gly Ser Gly
       450                 455                 460
   Gln Pro Arg Glu Pro Gln Val Tyr Thr Leu Pro Pro Ser Arg Glu Glu
   465                 470                 475                 480
   Met Thr Lys Asn Gln Val Ser Leu Thr Cys Leu Val Lys Gly Phe Tyr
                   485                 490                 495
   Pro Ser Asp Ile Ala Val Glu Trp Glu Ser Asn Gly Gln Pro Glu Asn
   500 505 510
   Asn Tyr Lys Thr Thr Pro Pro Val Leu Asp Ser Asp Gly Ser Phe Phe
   515 520 525
   Leu Tyr Ser Lys Leu Thr Val Asp Lys Ser Arg Trp Gln Gln Gly Asn
   530 535 540
   Val Phe Ser Cys Ser Val Met His Glu Ala Leu His Asn His Tyr Thr
   545 550 555 560
   Gln Lys Ser Leu Ser Leu Ser Pro Gly Ser Thr Gly Ser Glu Val Gln
   565 570 575
   Leu Gln Gln Ser Gly Pro Glu Leu Val Lys Pro Gly Ala Ser Val Lys
   580 585 590
   Ile Ser Cys Lys Thr Ser Gly Tyr Thr Phe Thr Glu Tyr Thr Met His
   595 600 605
   Trp Val Lys Gln Ser His Gly Lys Ser Leu Glu Trp Ile Gly Gly Ile
   610 615 620
   Ser Pro Asn Ile Gly Gly Thr Ser Tyr Asn Gln Lys Phe Lys Gly Lys
   625 630 635 640
   Ala Thr Leu Thr Val Asp Lys Ser Ser Ser Thr Ala Tyr Met Glu Leu
   645 650 655
   Arg Ser Leu Thr Ser Glu Asp Ser Ala Val Tyr Tyr Cys Ala Arg Arg
   660 665 670
   Gly Gly Ser Phe Asp Tyr Trp Gly Gln Gly Thr Thr Leu Thr Val Ser
   675 680 685
   Ser Arg Thr Val Ala Ala Pro Ser Val Phe Ile Phe Pro Pro Ser Asp
   690 695 700
   Glu Gln Leu Lys Ser Gly Thr Ala Ser Val Val Cys Leu Leu Asn Asn
   705 710 715 720
   Phe Tyr Pro Arg Glu Ala Lys Val Gln Trp Lys Val Asp Asn Ala Leu
   725 730 735
   Gln Ser Gly Asn Ser Gln Glu Ser Val Thr Glu Gln Asp Ser Lys Asp
   740 745 750
   Ser Thr Tyr Ser Leu Ser Ser Thr Leu Thr Leu Ser Lys Ala Asp Tyr
   755 760 765
   Glu Lys His Lys Val Tyr Ala Cys Glu Val Thr His Gln Gly Leu Ser
   770 775 780
   Ser Pro Val Thr Lys Ser Phe Asn Arg Gly Glu Cys
   785 790 795
<210> 17
   <211> 218
   <212> PRT
   <213> artificial
<220>
   <223> chimeric mus musculus homo sapiens
<400> 17
   Asp Ile Gln Leu Thr Gln Ser Pro Ala Ser Leu Ala Val Ser Leu Gly
   1 5 10 15
   Gln Arg Ala Thr Ile Ser Cys Lys Ala Ser Gln Ser Val Asp Tyr Asp
   20 25 30
   Gly Asp Ser Tyr Leu Asn Trp Tyr Gln Gln Ile Pro Gly Gln Pro Pro
   35 40 45
   Lys Leu Leu Ile Tyr Asp Ala Ser Asn Leu Val Ser Gly Ile Pro Pro
   50 55 60
   Arg Phe Ser Gly Ser Gly Ser Gly Thr Asp Phe Thr Leu Asn Ile His
   65 70 75 80
   Pro Val Glu Lys Val Asp Ala Ala Thr Tyr His Cys Gln Gln Ser Thr
   85 90 95
   Glu Asp Pro Trp Thr Phe Gly Gly Gly Thr Lys Leu Glu Ile Lys Arg
   100 105 110
   Thr Val Ala Ala Pro Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 18
   <211> 819
   <212> PRT
   <213> artificial
<220>
   <223> Chimeric mus musculus homo sapiens
<400> 18
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Thr Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Arg Val Glu Pro
       210                 215                 220
   Lys Ser Cys Asp Lys Thr His Thr Cys Pro Pro Cys Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Ser
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
   405 410 415
   Leu Thr Val Asp Lys Ser Arg Trp Gln Gln Gly Asn Val Phe Ser Cys
   420 425 430
   Ser Val Met His Glu Ala Leu His Asn His Tyr Thr Gln Lys Ser Leu
   435 440 445
   Ser Leu Ser Pro Gly Gly Gly Gly Gly Ser Gly Gly Gly Gly Ser Gly
   450 455 460
   Gly Gly Gly Ser Gly Gln Pro Arg Glu Pro Gln Val Tyr Thr Leu Pro
   465 470 475 480
   Pro Ser Arg Glu Glu Met Thr Lys Asn Gln Val Ser Leu Thr Cys Leu
   485 490 495
   Val Lys Gly Phe Tyr Pro Ser Asp Ile Ala Val Glu Trp Glu Ser Asn
   500 505 510
   Gly Gln Pro Glu Asn Asn Tyr Lys Thr Thr Pro Pro Val Leu Asp Ser
   515 520 525
   Asp Gly Ser Phe Phe Leu Tyr Ser Lys Leu Thr Val Asp Lys Ser Arg
   530 535 540
   Trp Gln Gln Gly Asn Val Phe Ser Cys Ser Val Met His Glu Ala Leu
   545 550 555 560
   His Asn His Tyr Thr Gln Lys Ser Leu Ser Leu Ser Pro Gly Ser Thr
   565 570 575
   Gly Ser Glu Val Gln Leu Gln Gln Ser Gly Pro Glu Leu Val Lys Pro
   580 585 590
   Gly Ala Ser Val Lys Ile Ser Cys Lys Thr Ser Gly Tyr Thr Phe Thr
   595 600 605
   Glu Tyr Thr Met His Trp Val Lys Gln Ser His Gly Lys Ser Leu Glu
   610 615 620
   Trp Ile Gly Gly Ile Ser Pro Asn Ile Gly Gly Thr Ser Tyr Asn Gln
   625 630 635 640
   Lys Phe Lys Gly Lys Ala Thr Leu Thr Val Asp Lys Ser Ser Ser Thr
                   645                 650                 655
   Ala Tyr Met Glu Leu Arg Ser Leu Thr Ser Glu Asp Ser Ala Val Tyr
               660                 665                 670
   Tyr Cys Ala Arg Arg Gly Gly Ser Phe Asp Tyr Trp Gly Gln Gly Thr
           675                 680                 685
   Thr Leu Thr Val Ser Ser Val Glu Gly Gly Ser Gly Gly Ser Gly Gly
       690                 695                 700
   Ser Gly Gly Ser Gly Gly Val Asp Asp Ile Val Met Thr Gln Ser Pro
   705                 710                 715                 720
   Ala Thr Leu Ser Val Thr Pro Gly Asp Arg Val Ser Leu Ser Cys Arg
                   725                 730                 735
   Ala Ser Gln Ser Ile Ser Asp Tyr Leu His Trp Tyr Gln Gln Lys Ser
               740                 745                 750
   His Glu Ser Pro Arg Leu Leu Ile Lys Tyr Ala Ser Gln Ser Ile Ser
           755                 760                 765
   Gly Ile Pro Ser Arg Phe Ser Gly Ser Gly Ser Gly Ser Asp Phe Thr
       770                 775                 780
   Leu Ser Ile Asn Ser Val Glu Pro Glu Asp Val Gly Val Tyr Tyr Cys
   785                 790                 795                 800
   Gln Asn Gly His Ser Phe Pro Leu Thr Phe Gly Ala Gly Thr Lys Leu
                   805                 810                 815
   Glu Leu Lys
<210> 19
   <211> 819
   <212> PRT
   <213> artificial
<220>
   <223> Chimeric mus musculus homo sapiens
<400> 19
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Thr Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Arg Val Glu Pro
       210                 215                 220
   Lys Ser Cys Asp Lys Thr His Thr Ser Pro Pro Ser Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Ser
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
                   405                 410                 415
   Leu Thr Val Asp Lys Ser Arg Trp Gln Gln Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Pro Gly Gly Gly Gly Gly Ser Gly Gly Gly Gly Ser Gly
       450                 455                 460
   Gly Gly Gly Ser Gly Gln Pro Arg Glu Pro Gln Val Tyr Thr Leu Pro
   465                 470                 475                 480
   Pro Ser Arg Glu Glu Met Thr Lys Asn Gln Val Ser Leu Thr Cys Leu
                   485                 490                 495
   Val Lys Gly Phe Tyr Pro Ser Asp Ile Ala Val Glu Trp Glu Ser Asn
               500                 505                 510
   Gly Gln Pro Glu Asn Asn Tyr Lys Thr Thr Pro Pro Val Leu Asp Ser
           515                 520                 525
   Asp Gly Ser Phe Phe Leu Tyr Ser Lys Leu Thr Val Asp Lys Ser Arg
       530                 535                 540
   Trp Gln Gln Gly Asn Val Phe Ser Cys Ser Val Met His Glu Ala Leu
   545                 550                 555                 560
   His Asn His Tyr Thr Gln Lys Ser Leu Ser Leu Ser Pro Gly Ser Thr
                   565                 570                 575
   Gly Ser Glu Val Gln Leu Gln Gln Ser Gly Pro Glu Leu Val Lys Pro
               580                 585                 590
   Gly Ala Ser Val Lys Ile Ser Cys Lys Thr Ser Gly Tyr Thr Phe Thr
           595                 600                 605
   Glu Tyr Thr Met His Trp Val Lys Gln Ser His Gly Lys Ser Leu Glu
       610                 615                 620
   Trp Ile Gly Gly Ile Ser Pro Asn Ile Gly Gly Thr Ser Tyr Asn Gln
   625                 630                 635                 640
   Lys Phe Lys Gly Lys Ala Thr Leu Thr Val Asp Lys Ser Ser Ser Thr
                   645                 650                 655
   Ala Tyr Met Glu Leu Arg Ser Leu Thr Ser Glu Asp Ser Ala Val Tyr
               660                 665                 670
   Tyr Cys Ala Arg Arg Gly Gly Ser Phe Asp Tyr Trp Gly Gln Gly Thr
           675                 680                 685
   Thr Leu Thr Val Ser Ser Val Glu Gly Gly Ser Gly Gly Ser Gly Gly
       690                 695                 700
   Ser Gly Gly Ser Gly Gly Val Asp Asp Ile Val Met Thr Gln Ser Pro
   705                 710                 715                 720
   Ala Thr Leu Ser Val Thr Pro Gly Asp Arg Val Ser Leu Ser Cys Arg
                   725                 730                 735
   Ala Ser Gln Ser Ile Ser Asp Tyr Leu His Trp Tyr Gln Gln Lys Ser
               740                 745                 750
   His Glu Ser Pro Arg Leu Leu Ile Lys Tyr Ala Ser Gln Ser Ile Ser
           755                 760                 765
   Gly Ile Pro Ser Arg Phe Ser Gly Ser Gly Ser Gly Ser Asp Phe Thr
       770                 775                 780
   Leu Ser Ile Asn Ser Val Glu Pro Glu Asp Val Gly Val Tyr Tyr Cys
   785 790 795 800
   Gln Asn Gly His Ser Phe Pro Leu Thr Phe Gly Ala Gly Thr Lys Leu
   805 810 815
   Glu Leu Lys
<210> 20
   <211> 815
   <212> PRT
   <213> artificial
<220>
   <223> Chimeric mus musculus homo sapiens
<400> 20
   Gln Val Gln Leu Gln Gln Ser Gly Ala Glu Leu Val Arg Pro Gly Ser
   1 5 10 15
   Ser Val Lys Ile Ser Cys Lys Ala Ser Gly Tyr Ala Phe Ser Ser Tyr
   20 25 30
   Trp Met Asn Trp Val Lys Gln Arg Pro Gly Gln Gly Leu Glu Trp Ile
   35 40 45
   Gly Gln Ile Trp Pro Gly Asp Gly Asp Thr Asn Tyr Asn Gly Lys Phe
   50 55 60
   Lys Gly Lys Ala Thr Leu Thr Ala Asp Glu Ser Ser Ser Thr Ala Tyr
   65 70 75 80
   Met Gln Leu Ser Ser Leu Ala Ser Glu Asp Ser Ala Val Tyr Phe Cys
   85 90 95
   Ala Arg Arg Glu Thr Thr Thr Val Gly Arg Tyr Tyr Tyr Ala Met Asp
   100 105 110
   Tyr Trp Gly Gln Gly Thr Thr Val Thr Val Ser Ser Ala Ser Thr Lys
   115 120 125
   Gly Pro Ser Val Phe Pro Leu Ala Pro Ser Ser Lys Ser Thr Ser Gly
   130 135 140
   Gly Thr Ala Ala Leu Gly Cys Leu Val Lys Asp Tyr Phe Pro Glu Pro
   145 150 155 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Arg Val Glu Pro
       210                 215                 220
   Lys Ser Cys Gly Gly Gly Ser Ser Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ser Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Gly Gly Gly Gly Ser Gly Gly Gly Gly Ser Gly Gly Gly Gly Ser
       450                 455                 460
   Gly Gln Pro Arg Glu Pro Gln Val Tyr Thr Leu Pro Pro Ser Arg Glu
   465                 470                 475                 480
   Glu Met Thr Lys Asn Gln Val Ser Leu Thr Cys Leu Val Lys Gly Phe
                   485                 490                 495
   Tyr Pro Ser Asp Ile Ala Val Glu Trp Glu Ser Asn Gly Gln Pro Glu
               500                 505                 510
   Asn Asn Tyr Lys Thr Thr Pro Pro Val Leu Asp Ser Asp Gly Ser Phe
           515                 520                 525
   Phe Leu Tyr Ser Lys Leu Thr Val Asp Lys Ser Arg Trp Gln Gln Gly
       530                 535                 540
   Asn Val Phe Ser Cys Ser Val Met His Glu Ala Leu His Asn His Tyr
   545                 550                 555                 560
   Thr Gln Lys Ser Leu Ser Leu Ser Pro Gly Ser Thr Gly Ser Glu Val
                   565                 570                 575
   Gln Leu Gln Gln Ser Gly Pro Glu Leu Val Lys Pro Gly Ala Ser Val
               580                 585                 590
   Lys Ile Ser Cys Lys Thr Ser Gly Tyr Thr Phe Thr Glu Tyr Thr Met
           595                 600                 605
   His Trp Val Lys Gln Ser His Gly Lys Ser Leu Glu Trp Ile Gly Gly
       610                 615                 620
   Ile Ser Pro Asn Ile Gly Gly Thr Ser Tyr Asn Gln Lys Phe Lys Gly
   625                 630                 635                 640
   Lys Ala Thr Leu Thr Val Asp Lys Ser Ser Ser Thr Ala Tyr Met Glu
                   645                 650                 655
   Leu Arg Ser Leu Thr Ser Glu Asp Ser Ala Val Tyr Tyr Cys Ala Arg
               660                 665                 670
   Arg Gly Gly Ser Phe Asp Tyr Trp Gly Gln Gly Thr Thr Leu Thr Val
   675 680 685
   Ser Ser Val Glu Gly Gly Ser Gly Gly Ser Gly Gly Ser Gly Gly Ser
   690 695 700
   Gly Gly Val Asp Asp Ile Val Met Thr Gln Ser Pro Ala Thr Leu Ser
   705 710 715 720
   Val Thr Pro Gly Asp Arg Val Ser Leu Ser Cys Arg Ala Ser Gln Ser
   725 730 735
   Ile Ser Asp Tyr Leu His Trp Tyr Gln Gln Lys Ser His Glu Ser Pro
   740 745 750
   Arg Leu Leu Ile Lys Tyr Ala Ser Gln Ser Ile Ser Gly Ile Pro Ser
   755 760 765
   Arg Phe Ser Gly Ser Gly Ser Gly Ser Asp Phe Thr Leu Ser Ile Asn
   770 775 780
   Ser Val Glu Pro Glu Asp Val Gly Val Tyr Tyr Cys Gln Asn Gly His
   785 790 795 800
   Ser Phe Pro Leu Thr Phe Gly Ala Gly Thr Lys Leu Glu Leu Lys
   805 810 815
<210> 21
   <211> 819
   <212> PRT
   <213> artificial
<220>
   <223> Chimeric mus musculus homo sapiens
<400> 21
   Asp Ile Gln Leu Thr Gln Ser Pro Ala Ser Leu Ala Val Ser Leu Gly
   1 5 10 15
   Gln Arg Ala Thr Ile Ser Cys Lys Ala Ser Gln Ser Val Asp Tyr Asp
   20 25 30
   Gly Asp Ser Tyr Leu Asn Trp Tyr Gln Gln Ile Pro Gly Gln Pro Pro
   35 40 45
   Lys Leu Leu Ile Tyr Asp Ala Ser Asn Leu Val Ser Gly Ile Pro Pro
   50 55 60
   Arg Phe Ser Gly Ser Gly Ser Gly Thr Asp Phe Thr Leu Asn Ile His
   65 70 75 80
   Pro Val Glu Lys Val Asp Ala Ala Thr Tyr His Cys Gln Gln Ser Thr
   85 90 95
   Glu Asp Pro Trp Thr Phe Gly Gly Gly Thr Lys Leu Glu Ile Lys Gly
   100 105 110
   Gly Gly Gly Ser Gly Gly Gly Gly Ser Gly Gly Gly Gly Ser Gln Val
   115 120 125
   Gln Leu Gln Gln Ser Gly Ala Glu Leu Val Arg Pro Gly Ser Ser Val
   130 135 140
   Lys Ile Ser Cys Lys Ala Ser Gly Tyr Ala Phe Ser Ser Tyr Trp Met
   145 150 155 160
   Asn Trp Val Lys Gln Arg Pro Gly Gln Gly Leu Glu Trp Ile Gly Gln
   165 170 175
   Ile Trp Pro Gly Asp Gly Asp Thr Asn Tyr Asn Gly Lys Phe Lys Gly
   180 185 190
   Lys Ala Thr Leu Thr Ala Asp Glu Ser Ser Ser Thr Ala Tyr Met Gln
   195 200 205
   Leu Ser Ser Leu Ala Ser Glu Asp Ser Ala Val Tyr Phe Cys Ala Arg
   210 215 220
   Arg Glu Thr Thr Thr Val Gly Arg Tyr Tyr Tyr Ala Met Asp Tyr Trp
   225 230 235 240
   Gly Gln Gly Thr Thr Val Thr Val Ser Ser Gly Gly Gly Ser Ser Ala
   245 250 255
   Pro Glu Leu Leu Gly Gly Pro Ser Val Phe Leu Phe Pro Pro Lys Pro
   260 265 270
   Lys Asp Thr Leu Met Ile Ser Arg Thr Pro Glu Val Thr Cys Val Val
   275 280 285
   Val Asp Val Ser His Glu Asp Pro Glu Val Lys Phe Asn Trp Tyr Val
   290 295 300
   Asp Gly Val Glu Val His Asn Ala Lys Thr Lys Pro Arg Glu Glu Gln
   305 310 315 320
   Tyr Ser Ser Thr Tyr Arg Val Val Ser Val Leu Thr Val Leu His Gln
                   325                 330                 335
   Asp Trp Leu Asn Gly Lys Glu Tyr Lys Cys Lys Val Ser Asn Lys Ala
               340                 345                 350
   Leu Pro Ala Pro Ile Glu Lys Thr Ile Ser Lys Ala Lys Gly Gln Pro
           355                 360                 365
   Arg Glu Pro Gln Val Tyr Thr Leu Pro Pro Ser Arg Glu Glu Met Thr
       370                 375                 380
   Lys Asn Gln Val Ser Leu Thr Cys Leu Val Lys Gly Phe Tyr Pro Ser
   385                 390                 395                 400
   Asp Ile Ala Val Glu Trp Glu Ser Asn Gly Gln Pro Glu Asn Asn Tyr
                   405                 410                 415
   Lys Thr Thr Pro Pro Val Leu Asp Ser Asp Gly Ser Phe Phe Leu Tyr
               420                 425                 430
   Ser Lys Leu Thr Val Asp Lys Ser Arg Trp Gln Gln Gly Asn Val Phe
           435                 440                 445
   Ser Cys Ser Val Met His Glu Ala Leu His Asn His Tyr Thr Gln Lys
       450                 455                 460
   Ser Leu Ser Leu Ser Pro Gly Gly Gly Gly Gly Ser Gly Gly Gly Gly
   465                 470                 475                 480
   Ser Gly Gly Gly Gly Ser Gly Gln Pro Arg Glu Pro Gln Val Tyr Thr
                   485                 490                 495
   Leu Pro Pro Ser Arg Glu Glu Met Thr Lys Asn Gln Val Ser Leu Thr
               500                 505                 510
   Cys Leu Val Lys Gly Phe Tyr Pro Ser Asp Ile Ala Val Glu Trp Glu
           515                 520                 525
   Ser Asn Gly Gln Pro Glu Asn Asn Tyr Lys Thr Thr Pro Pro Val Leu
       530                 535                 540
   Asp Ser Asp Gly Ser Phe Phe Leu Tyr Ser Lys Leu Thr Val Asp Lys
   545                 550                 555                 560
   Ser Arg Trp Gln Gln Gly Asn Val Phe Ser Cys Ser Val Met His Glu
   565 570 575
   Ala Leu His Asn His Tyr Thr Gln Lys Ser Leu Ser Leu Ser Pro Gly
   580 585 590
   Ser Thr Gly Ser Glu Val Gln Leu Gln Gln Ser Gly Pro Glu Leu Val
   595 600 605
   Lys Pro Gly Ala Ser Val Lys Ile Ser Cys Lys Thr Ser Gly Tyr Thr
   610 615 620
   Phe Thr Glu Tyr Thr Met His Trp Val Lys Gln Ser His Gly Lys Ser
   625 630 635 640
   Leu Glu Trp Ile Gly Gly Ile Ser Pro Asn Ile Gly Gly Thr Ser Tyr
   645 650 655
   Asn Gln Lys Phe Lys Gly Lys Ala Thr Leu Thr Val Asp Lys Ser Ser
   660 665 670
   Ser Thr Ala Tyr Met Glu Leu Arg Ser Leu Thr Ser Glu Asp Ser Ala
   675 680 685
   Val Tyr Tyr Cys Ala Arg Arg Gly Gly Ser Phe Asp Tyr Trp Gly Gln
   690 695 700
   Gly Thr Thr Leu Thr Val Ser Ser Arg Thr Val Ala Ala Pro Ser Val
   705 710 715 720
   Phe Ile Phe Pro Pro Ser Asp Glu Gln Leu Lys Ser Gly Thr Ala Ser
   725 730 735
   Val Val Cys Leu Leu Asn Asn Phe Tyr Pro Arg Glu Ala Lys Val Gln
   740 745 750
   Trp Lys Val Asp Asn Ala Leu Gln Ser Gly Asn Ser Gln Glu Ser Val
   755 760 765
   Thr Glu Gln Asp Ser Lys Asp Ser Thr Tyr Ser Leu Ser Ser Thr Leu
   770 775 780
   Thr Leu Ser Lys Ala Asp Tyr Glu Lys His Lys Val Tyr Ala Cys Glu
   785 790 795 800
   Val Thr His Gln Gly Leu Ser Ser Pro Val Thr Lys Ser Phe Asn Arg
   805 810 815
   Gly Glu Cys
<210> 22
   <211> 214
   <212> PRT
   <213> artificial
<220>
   <223> Chimeric mus musculus homo sapiens
<400> 22
   Asp Ile Val Met Thr Gln Ser Pro Ala Thr Leu Ser Val Thr Pro Gly
   1               5                   10                  15
   Asp Arg Val Ser Leu Ser Cys Arg Ala Ser Gln Ser Ile Ser Asp Tyr
               20                  25                  30
   Leu His Trp Tyr Gln Gln Lys Ser His Glu Ser Pro Arg Leu Leu Ile
           35                  40                  45
   Lys Tyr Ala Ser Gln Ser Ile Ser Gly Ile Pro Ser Arg Phe Ser Gly
       50                  55                  60
   Ser Gly Ser Gly Ser Asp Phe Thr Leu Ser Ile Asn Ser Val Glu Pro
   65                  70                  75                  80
   Glu Asp Val Gly Val Tyr Tyr Cys Gln Asn Gly His Ser Phe Pro Leu
                   85                  90                  95
   Thr Phe Gly Ala Gly Thr Lys Leu Glu Leu Lys Ala Ser Thr Lys Gly
               100                 105                 110
   Pro Ser Val Phe Pro Leu Ala Pro Ser Ser Lys Ser Thr Ser Gly Gly
           115                 120                 125
   Thr Ala Ala Leu Gly Cys Leu Val Lys Asp Tyr Phe Pro Glu Pro Val
       130                 135                 140
   Thr Val Ser Trp Asn Ser Gly Ala Leu Thr Ser Gly Val His Thr Phe
   145                 150                 155                 160
   Pro Ala Val Leu Gln Ser Ser Gly Leu Tyr Ser Leu Ser Ser Val Val
                   165                 170                 175
   Thr Val Pro Ser Ser Ser Leu Gly Thr Gln Thr Tyr Ile Cys Asn Val
               180                 185                 190
   Asn His Lys Pro Ser Asn Thr Lys Val Asp Lys Arg Val Glu Pro Lys
                 195                 200                 205
   Ser Cys Asp Lys Thr His
       210
<210> 23
   <211> 819
   <212> PRT
   <213> artificial
<220>
   <223> Chimeric mus musculus homo sapiens
<400> 23
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ser Gln Val
           115                 120                 125
   Gln Leu Gln Gln Ser Gly Ala Glu Leu Val Arg Pro Gly Ser Ser Val
       130                 135                 140
   Lys Ile Ser Cys Lys Ala Ser Gly Tyr Ala Phe Ser Ser Tyr Trp Met
   145                 150                 155                 160
   Asn Trp Val Lys Gln Arg Pro Gly Gln Gly Leu Glu Trp Ile Gly Gln
                   165                 170                 175
   Ile Trp Pro Gly Asp Gly Asp Thr Asn Tyr Asn Gly Lys Phe Lys Gly
               180                 185                 190
   Lys Ala Thr Leu Thr Ala Asp Glu Ser Ser Ser Thr Ala Tyr Met Gln
           195                 200                 205
   Leu Ser Ser Leu Ala Ser Glu Asp Ser Ala Val Tyr Phe Cys Ala Arg
       210                 215                 220
   Arg Glu Thr Thr Thr Val Gly Arg Tyr Tyr Tyr Ala Met Asp Tyr Trp
   225                 230                 235                 240
   Gly Gln Gly Thr Thr Val Thr Val Ser Ser Gly Gly Gly Ser Ser Ala
                   245                 250                 255
   Pro Glu Leu Leu Gly Gly Pro Ser Val Phe Leu Phe Pro Pro Lys Pro
               260                 265                 270
   Lys Asp Thr Leu Met Ile Ser Arg Thr Pro Glu Val Thr Cys Val Val
           275                 280                 285
   Val Asp Val Ser His Glu Asp Pro Glu Val Lys Phe Asn Trp Tyr Val
       290                 295                 300
   Asp Gly Val Glu Val His Asn Ala Lys Thr Lys Pro Arg Glu Glu Gln
   305                 310                 315                 320
   Tyr Ser Ser Thr Tyr Arg Val Val Ser Val Leu Thr Val Leu His Gln
                   325                 330                 335
   Asp Trp Leu Asn Gly Lys Glu Tyr Lys Cys Lys Val Ser Asn Lys Ala
               340                 345                 350
   Leu Pro Ala Pro Ile Glu Lys Thr Ile Ser Lys Ala Lys Gly Gln Pro
           355                 360                 365
   Arg Glu Pro Gln Val Tyr Thr Leu Pro Pro Ser Arg Glu Glu Met Thr
       370                 375                 380
   Lys Asn Gln Val Ser Leu Thr Cys Leu Val Lys Gly Phe Tyr Pro Ser
   385                 390                 395                 400
   Asp Ile Ala Val Glu Trp Glu Ser Asn Gly Gln Pro Glu Asn Asn Tyr
                   405                 410                 415
   Lys Thr Thr Pro Pro Val Leu Asp Ser Asp Gly Ser Phe Phe Leu Tyr
               420                 425                 430
   Ser Lys Leu Thr Val Asp Lys Ser Arg Trp Gln Gln Gly Asn Val Phe
           435                 440                 445
   Ser Cys Ser Val Met His Glu Ala Leu His Asn His Tyr Thr Gln Lys
       450                 455                 460
   Ser Leu Ser Leu Ser Pro Gly Gly Gly Gly Gly Ser Gly Gly Gly Gly
   465                 470                 475                 480
   Ser Gly Gly Gly Gly Ser Gly Gln Pro Arg Glu Pro Gln Val Tyr Thr
                   485                 490                 495
   Leu Pro Pro Ser Arg Glu Glu Met Thr Lys Asn Gln Val Ser Leu Thr
               500                 505                 510
   Cys Leu Val Lys Gly Phe Tyr Pro Ser Asp Ile Ala Val Glu Trp Glu
           515                 520                 525
   Ser Asn Gly Gln Pro Glu Asn Asn Tyr Lys Thr Thr Pro Pro Val Leu
       530                 535                 540
   Asp Ser Asp Gly Ser Phe Phe Leu Tyr Ser Lys Leu Thr Val Asp Lys
   545                 550                 555                 560
   Ser Arg Trp Gln Gln Gly Asn Val Phe Ser Cys Ser Val Met His Glu
                   565                 570                 575
   Ala Leu His Asn His Tyr Thr Gln Lys Ser Leu Ser Leu Ser Pro Gly
               580                 585                 590
   Ser Thr Gly Ser Glu Val Gln Leu Gln Gln Ser Gly Pro Glu Leu Val
           595                 600                 605
   Lys Pro Gly Ala Ser Val Lys Ile Ser Cys Lys Thr Ser Gly Tyr Thr
       610                 615                 620
   Phe Thr Glu Tyr Thr Met His Trp Val Lys Gln Ser His Gly Lys Ser
   625                 630                 635                 640
   Leu Glu Trp Ile Gly Gly Ile Ser Pro Asn Ile Gly Gly Thr Ser Tyr
                   645                 650                 655
   Asn Gln Lys Phe Lys Gly Lys Ala Thr Leu Thr Val Asp Lys Ser Ser
               660                 665                 670
   Ser Thr Ala Tyr Met Glu Leu Arg Ser Leu Thr Ser Glu Asp Ser Ala
           675                 680                 685
   Val Tyr Tyr Cys Ala Arg Arg Gly Gly Ser Phe Asp Tyr Trp Gly Gln
       690                 695                 700
   Gly Thr Thr Leu Thr Val Ser Ser Ala Ser Thr Lys Gly Pro Ser Val
   705                 710                 715                 720
   Phe Pro Leu Ala Pro Ser Ser Lys Ser Thr Ser Gly Gly Thr Ala Ala
                   725                 730                 735
   Leu Gly Cys Leu Val Lys Asp Tyr Phe Pro Glu Pro Val Thr Val Ser
               740                 745                 750
   Trp Asn Ser Gly Ala Leu Thr Ser Gly Val His Thr Phe Pro Ala Val
           755                 760                 765
   Leu Gln Ser Ser Gly Leu Tyr Ser Leu Ser Ser Val Val Thr Val Pro
       770                 775                 780
   Ser Ser Ser Leu Gly Thr Gln Thr Tyr Ile Cys Asn Val Asn His Lys
   785                 790                 795                 800
   Pro Ser Asn Thr Lys Val Asp Lys Arg Val Glu Pro Lys Ser Cys Asp
                   805                 810                 815
   Lys Thr His
<210> 24
   <211> 214
   <212> PRT
   <213> artificial
<220>
   <223> Chimeric mus musculus homo sapiens
<400> 24
   Asp Ile Val Met Thr Gln Ser Pro Ala Thr Leu Ser Val Thr Pro Gly
   1               5                   10                  15
   Asp Arg Val Ser Leu Ser Cys Arg Ala Ser Gln Ser Ile Ser Asp Tyr
               20                  25                  30
   Leu His Trp Tyr Gln Gln Lys Ser His Glu Ser Pro Arg Leu Leu Ile
           35                  40                  45
   Lys Tyr Ala Ser Gln Ser Ile Ser Gly Ile Pro Ser Arg Phe Ser Gly
       50                  55                  60
   Ser Gly Ser Gly Ser Asp Phe Thr Leu Ser Ile Asn Ser Val Glu Pro
   65                  70                  75                  80
   Glu Asp Val Gly Val Tyr Tyr Cys Gln Asn Gly His Ser Phe Pro Leu
                   85                  90                  95
   Thr Phe Gly Ala Gly Thr Lys Leu Glu Leu Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 25
   <211> 218
   <212> PRT
   <213> artificial
<220>
   <223> Chimeric mus musculus homo sapiens
<400> 25
   Asp Ile Gln Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 26
   <211> 792
   <212> PRT
   <213> artificial
<220>
   <223> Chimeric mus musculus homo sapiens
<400> 26
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Thr Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Arg Val Glu Pro
       210                 215                 220
   Lys Ser Cys Asp Lys Thr His Thr Ser Pro Pro Ser Pro Ala Pro Glu
   225                 230                 235                 240
   Leu Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser His Glu Asp Pro Glu Val Lys Phe Asn Trp Tyr Val Asp Gly
           275                 280                 285
   Val Glu Val His Asn Ala Lys Thr Lys Pro Arg Glu Glu Gln Tyr Ser
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Ala Leu Pro
                   325                 330                 335
   Ala Pro Ile Glu Lys Thr Ile Ser Lys Ala Lys Gly Gln Pro Arg Glu
               340                 345                 350
   Pro Gln Val Tyr Thr Leu Pro Pro Ser Arg Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Lys
                   405                 410                 415
   Leu Thr Val Asp Lys Ser Arg Trp Gln Gln Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Pro Gly Gly Gly Gly Gly Ser Gly Gly Gly Gly Ser Gly
       450                 455                 460
   Gly Gly Gly Ser Gly Gln Pro Arg Glu Pro Gln Val Tyr Thr Leu Pro
   465                 470                 475                 480
   Pro Ser Arg Glu Glu Met Thr Lys Asn Gln Val Ser Leu Thr Cys Leu
                   485                 490                 495
   Val Lys Gly Phe Tyr Pro Ser Asp Ile Ala Val Glu Trp Glu Ser Asn
               500                 505                 510
   Gly Gln Pro Glu Asn Asn Tyr Lys Thr Thr Pro Pro Val Leu Asp Ser
           515                 520                 525
   Asp Gly Ser Phe Phe Leu Tyr Ser Lys Leu Thr Val Asp Lys Ser Arg
       530                 535                 540
   Trp Gln Gln Gly Asn Val Phe Ser Cys Ser Val Met His Glu Ala Leu
   545 550 555 560
   His Asn His Tyr Thr Gln Lys Ser Leu Ser Leu Ser Pro Gly Ser Thr
   565 570 575
   Gly Ser Asp Ile Val Met Thr Gln Ser Pro Ala Thr Leu Ser Val Thr
   580 585 590
   Pro Gly Asp Arg Val Ser Leu Ser Cys Arg Ala Ser Gln Ser Ile Ser
   595 600 605
   Asp Tyr Leu His Trp Tyr Gln Gln Lys Ser His Glu Ser Pro Arg Leu
   610 615 620
   Leu Ile Lys Tyr Ala Ser Gln Ser Ile Ser Gly Ile Pro Ser Arg Phe
   625 630 635 640
   Ser Gly Ser Gly Ser Gly Ser Asp Phe Thr Leu Ser Ile Asn Ser Val
   645 650 655
   Glu Pro Glu Asp Val Gly Val Tyr Tyr Cys Gln Asn Gly His Ser Phe
   660 665 670
   Pro Leu Thr Phe Gly Ala Gly Thr Lys Leu Glu Leu Lys Ala Ser Thr
   675 680 685
   Lys Gly Pro Ser Val Phe Pro Leu Ala Pro Ser Ser Lys Ser Thr Ser
   690 695 700
   Gly Gly Thr Ala Ala Leu Gly Cys Leu Val Lys Asp Tyr Phe Pro Glu
   705 710 715 720
   Pro Val Thr Val Ser Trp Asn Ser Gly Ala Leu Thr Ser Gly Val His
   725 730 735
   Thr Phe Pro Ala Val Leu Gln Ser Ser Gly Leu Tyr Ser Leu Ser Ser
   740 745 750
   Val Val Thr Val Pro Ser Ser Ser Leu Gly Thr Gln Thr Tyr Ile Cys
   755 760 765
   Asn Val Asn His Lys Pro Ser Asn Thr Lys Val Asp Lys Arg Val Glu
   770 775 780
   Pro Lys Ser Cys Asp Lys Thr His
   785 790
<210> 27
   <211> 223
   <212> PRT
   <213> artificial
<220>
   <223> Chimeric mus musculus homo sapiens
<400> 27
   Glu Val Gln Leu Gln Gln Ser Gly Pro Glu Leu Val Lys Pro Gly Ala
   1 5 10 15
   Ser Val Lys Ile Ser Cys Lys Thr Ser Gly Tyr Thr Phe Thr Glu Tyr
   20 25 30
   Thr Met His Trp Val Lys Gln Ser His Gly Lys Ser Leu Glu Trp Ile
   35 40 45
   Gly Gly Ile Ser Pro Asn Ile Gly Gly Thr Ser Tyr Asn Gln Lys Phe
   50 55 60
   Lys Gly Lys Ala Thr Leu Thr Val Asp Lys Ser Ser Ser Thr Ala Tyr
   65 70 75 80
   Met Glu Leu Arg Ser Leu Thr Ser Glu Asp Ser Ala Val Tyr Tyr Cys
   85 90 95
   Ala Arg Arg Gly Gly Ser Phe Asp Tyr Trp Gly Gln Gly Thr Thr Leu
   100 105 110
   Thr Val Ser Ser Arg Thr Val Ala Ala Pro Ser Val Phe Ile Phe Pro
   115 120 125
   Pro Ser Asp Glu Gln Leu Lys Ser Gly Thr Ala Ser Val Val Cys Leu
   130 135 140
   Leu Asn Asn Phe Tyr Pro Arg Glu Ala Lys Val Gln Trp Lys Val Asp
   145 150 155 160
   Asn Ala Leu Gln Ser Gly Asn Ser Gln Glu Ser Val Thr Glu Gln Asp
   165 170 175
   Ser Lys Asp Ser Thr Tyr Ser Leu Ser Ser Thr Leu Thr Leu Ser Lys
   180 185 190
   Ala Asp Tyr Glu Lys His Lys Val Tyr Ala Cys Glu Val Thr His Gln
   195 200 205
   Gly Leu Ser Ser Pro Val Thr Lys Ser Phe Asn Arg Gly Glu Cys
   210 215 220
<210> 28
   <211> 5
   <212> PRT
   <213> artificial
<220>
   <223> linker
<400> 28
   Gly Gly Gly Ser Ser
   1 5
<210> 29
   <211> 10
   <212> PRT
   <213> human
<400> 29
   Asp Lys Thr His Thr Cys Pro Pro Cys Pro
   1 5 10

## Description

### FIELD OF THE INVENTION

Monomeric Fc-domains are provided, as well as multimeric and single chain proteins comprising the monomeric Fc-domains. The proteins have utility in the treatment of disease. Included, inter alia, are multispecific polypeptides.

### BACKGROUND

Antibody fragments such as Fab, Fv, scFv, VH and VHH, and more new antibody fragment formats are under development that have decreased size and may provide improved tissue penetration, for example for treating solid tumors. New formats have also been developed for bispecific antibodies binding two different epitopes offering opportunities for increasing specificity, broadening potency, and utilizing novel mechanisms of action that cannot be achieved with a traditional monoclonal antibody. However, many of these formats have very short half-lives compared to full-size IgG.

The Fc domain increases the half-life of an IgG through its unique pH-dependent association with the neonatal Fc receptor (FcRn). After internalization, the Fc domain of IgG can bind to FcRn in the acidic environment of the endosome, so that the IgG is then cycled onto the cell surface and re-released into circulation. This biological system protects IgG from degradation and results in a long serum half-life. Fusions of an Fc domain and a therapeutic molecule have an extended half-life. In addition, since the Fc fragment of IgG consists of a tightly packed homodimer, two therapeutic proteins are present in each molecule. One approach to produce smaller monomeric Fc fusion involved introducing mutations into the CH3 domain to prevent CH3-CH3 homodimerization (see, e.g., WO2013/138643 and WO2011/063348). However, introduction of mutations involves risk of immunogenicity when administered to humans.

Dall'Acqua et al (1998, Biochemistry, 37, 9266-9273) describes the use of a single-chain CH3 dimer in order to study the interface of CH3 homodimers.

WO2008/012543 describes a single chain Fc polypeptide with two CH2-CH3 sections arranged so as to form a full dimeric Fc domain.

WO2008/131242 and WO2010/045261 describes proteins with full dimeric Fc domains formed from two CH2 and two CH3.

There remains therefore a need to produce new protein formats that permit the use of monomeric Fc domains.

### SUMMARY OF THE INVENTION

The scope of the present invention is defined by the claims and any information that does not fall within the claims is provided for information only.

The present disclosure arises from the discovery of a Fc-polypeptide construct with two immunoglobulin CH3 domains arranged in tandem that yields functional proteins in which the Fc domain remains monomeric and interacts with the neonatal Fc receptor (FcRn). The constructs permit a range of single chain and multi-chain (multimeric) proteins to be constructed, including but not limit to single chain and multimeric bispecific binding proteins, e.g., antibodies.

The two CH3 domains within a single polypeptide chain (referred to as "tandem CH3 domains") can be incorporated into any Fc-domain containing molecule, e.g. a molecule that comprises other amino acid sequences or protein domains on the same polypeptide chain and/or on additional polypeptide chains that multimerize with the tandem CH3 domain-containing polypeptide chain. While the Fc domain remains monomeric (e.g., not an Fc dimer formed from two Fc domains that undergo CH3-CH3 dimerization), the protein that contains the tandem CH3 domain may be monomeric or multimeric (containing more than one polypeptide chains, e.g., a dimer, trimer, tetramer, pentamer).

A tandem CH3 domain-containing polypeptide chain may typically comprise a CH2 domain linked to one of the two CH3 domains, and optionally further a protein domain of interest, e.g., an immunoglobulin variable region such as a heavy chain variable region (VH) and/or light chain variable region (VL), and/or generally any antigen binding domain. The domain can for example be fused directly to the CH2 or CH3 domain or via intervening sequence of amino acid residues such as a peptide linker, a hinge, a CH1 domain or a CK domain, and the like. Providing a suitable linker (e.g. any amino acid sequence or other flexible molecule) within the tandem CH3 domain between the two CH3 domains permits the CH3 domains to associate with one another by non-covalent interactions. The two CH3 domains are therefore not available for CH3-CH3 dimerization with other polypeptide chains, thereby avoiding homo-dimerization among CH3 domain-containing molecules. In one example, tandem CH3 domains are incorporated into a polypeptide that further comprises a CH2 domain, resulting in an Fc domain that remains monomeric. The single polypeptide chain having a monomeric Fc domain can be used as such or can be combined with further polypeptide chain to give rise to a multimeric polypeptide having a monomeric Fc domain.

These Fc-domain containing molecules disclosed herein have the advantage of accommodating native CH3 domains. The molecules can also be easily produced. They can furthermore harbor more than one functional protein domain, e.g. an antigen binding domain, permitting a wide range of Fc-domain containing molecules to be produced. In particular, bispecific antibodies can be produced that have a monomeric Fc domain that binds the neonatal Fc (FcRn) receptor.

In one aspect provided is a tandem CH3 domain. In one aspect provided is an Fc domain comprising a CH2 domain and the tandem CH3 domain. In one aspect provided is a polypeptide chain comprising the tandem CH3 domain or the Fc domain. In one aspect provided is a multimeric polypeptide comprising the polypeptide chain comprising such tandem CH3 domain. In one aspect, the two CH3 domains of the tandem CH3 domain are separated by a flexible peptide linker. In one aspect, the two CH3 domains of the tandem CH3 domain associate with one another via non-covalent interactions. In one aspect, the two CH3 domains associate with one another via the CH3 dimerization interface of each CH3 domain. In one aspect, the tandem CH3 domain is incapable of further CH3-CH3 dimerization (e.g., with a CH3 domain of a further polypeptide chain).

In one aspect, provided is a polypeptide chain comprising a first and a second CH3 domain, wherein the two CH3 domains associate with one another via non-covalent interactions. In one aspect, the two CH3 domains associate with one another via the CH3 dimerization interface of each CH3 domain. In one aspect, the polypeptide chain does not dimerize with another polypeptide chain comprising an Fc domain.

In one aspect, provided is a multimeric (e.g. a dimeric, trimeric, tetrameric, pentameric) protein having a monomeric Fc domain, the protein comprising a polypeptide chain comprising an Fc domain comprising a CH2 domain and a tandem CH3 domain. In one aspect, the two CH3 domains of the Fc domain associate with one another via non-covalent interactions.

In one aspect of any aspect herein, a first CH3 domain is connected to a second CH3 domain by a linker. The tandem CH3 domains can thus be placed on the same polypeptide chain so as to have the domain arrangement as follows:

- CH3 - linker - CH3 -

The linker will be a flexible linker (e.g. peptide linker). In one aspect the linker permits the CH3 domains to associate with one another by non-covalent interactions. In one aspect, the linker is a peptide linker having 10-50 amino acid residues. In one aspect, the linker has the formula (G₄S)ₓ. Optionally, x is 2, 3, 4, 5 or 6.

In any of the aspects, each CH3 domain is independently a human full-length and/or native CH3 domain, or a fragment or modified CH3 domain which retains a functional CH3 dimerization interface (e.g. thereof that retains the ability to undergo CH3-CH3 dimerization with another CH3 domain).

In any of the aspects herein, an exemplary CH3 domain for use in a tandem CH3 domain or Fc domain comprises an amino acid sequence of SEQ ID NO : 1, or a sequence at least 70%, 80%, 90%, 95% or 98% identical thereto.

In any of the aspects herein, an exemplary tandem CH3 domain comprises an amino acid sequence of SEQ ID NO : 2, or a sequence at least 70%, 80%, 90%, 95% or 98% identical thereto.

In one aspect provided is a polypeptide comprising a monomeric Fc domain comprising a native CH3 domain (i.e., which retains a functional CH3 dimerization interface). In one aspect the polypeptide is a monomeric polypeptide. In one aspect the polypeptide is a multimeric polypeptide comprising a monomeric Fc domain. In one aspect, the tandem CH3 domain is incapable of further CH3-CH3 dimerization (i.e. with a further CH3 domain of a polypeptide).

In one aspect of any aspect, provided is an isolated single chain polypeptide comprising a tandem CH3 domain. Also provided is a single chain Fc domain molecule comprising a tandem CH3 domain, such as a fusion protein including the monomeric Fc domain molecule or tandem CH3 domain. In one aspect, the Fc domain molecule comprises a soluble polypeptide fused to an Fc domain. In one aspect, the Fc domain molecule comprises an extracellular domain (ECD) of a cell surface receptor fused to an Fc domain, optionally via intervening sequences of amino acid residues (e.g. a linker, a protein domain, etc.). One example of Fc fusion polypeptide is a polypeptide with the domain arrangement, from N-terminus (left) to C-terminus (right):
(ECD) - CH2 - CH3 - linker - CH3.

In one aspect the fusion protein is a monomeric or multimeric antibody-derived polypeptide. These molecules include a further polypeptide fused at the N- or C- terminus of the Fc domain or CH3 domain. In some non-limiting examples, the heterologous protein is a protein of interest (shown as (P) in the domain arrangement below), an antigen binding domain (ABD), a immunoglobulin variable domain, a cytokine, or a receptor polypeptide. A protein (P) can be any desired sequence of amino acid residues, e.g. 1-20, 20-50, 10-100, 1-200, 1-500, or 20-500 amino acid residues. Tandem CH3 domains can be comprised in polypeptide chains having one the following domain arrangements, which polypeptide chains can remain monomeric or can associate with further polypeptide chains to form multimeric polypeptides with monomeric Fc domains:
(P) - CH2 - CH3 - linker - CH3,
(ABD) - CH2 - CH3 - linker - CH3, or
(scFv) - CH2 - CH3 - linker - CH3.

If desired, a heterologous polypeptide can be fused to both the N- and C- terminus of the Fc domain or CH3 domain, e.g. a tandem CH3 domains can be comprised in polypeptide chain together with a first and second protein of interest (shown as (P₁ and P₂) in the domain arrangement below), according to an exemplary domain arrangement:
(P₁) - CH2 - CH3 - linker - CH3 - (P₂).

Examples of multimeric antibody-derived polypeptides with monomeric Fc domains include polypeptides with domain arrangements below, where the polypeptide chains can associate with one another by CH1-CK heterodimerization (linkage shown between CH1 and CK), forming interchain non-covalent bonds, and optionally further disulfide bonds) between respective complementary CH1 and CK domains.

One example is shown as follows: wherein the variable region in one chain is a VH and the variable region in the other chain is a VL, such that the VH and VL form an antigen binding domain that binds an antigen of interest, and wherein the CH1 or CK constant region of one chain is a CH1 and the CH1 or CK constant region in the other chain is a CK, such that the CH1 and CK in the respective chains associate by non-covalent bonds (and optionally further disulfide bonds). Each polypeptide chain may optionally comprise one or more domains at its N- and/or C- terminus. In one aspect, a hinge domain is placed between the CH1 or CK domain and the CH2 domain.

Examples of protein domain arrangements include: or or or or or or or

Each polypeptide chain may optionally comprise one or more domains at its N- and/or C- terminus. In one aspect, a hinge domain is placed between the CH1 or CK domain and the CH2 domain. In one aspect of any polypeptide herein, a VH or VK domain, or a CH1 or CK (or Cλ) domain, will be fused to an Fc domain (e.g. or a CH2 domain thereof) via a hinge region.

The antibodies are also particularly well adapted for use as a multispecific (e.g. bispecific) binding protein. For example, the bispecific protein can bind a first antigen on a target cell to be eliminated and a second antigen on an immune effector cell (e.g. an NK cell and/or a T cell), where the effector cells are directed to the target cell, e.g. a cancer cell. The multispecific antibody retains FcRn binding yet lacks FcγR binding such that the multispecific antibody has attractive in vivo pharmacodynamics yet is selective for the particular effector cells of interest (i.e. the cells expressing the second antigen), thereby reducing toxicity mediated by immune cells other than those expressing the particular effector cell surface antigen to which the antibody binds. The multispecific polypeptide is capable, for example, of directing antigen-of-interest-expressing effector cells to lyse a target cell expressing a target antigen, e.g. cancer antigen, viral antigen, etc. The multispecific antibody is particularly effective when binding both effector cell surface protein and a second antigen (an antigen expressed by a target cell) in monovalent fashion.

In one aspect, provided is a multispecific (e.g. bispecific) polypeptide that binds a first and a second antigen each in monovalent fashion, comprising: a first antigen binding domain (ABD₁) that specifically binds to a first antigen of interest, a second antigen binding domain (ABD₂) that specifically binds a second antigen of interest, and a first and a second human CH3 domain separated by a flexible linker. In one aspect, the first and second human CH3 domain separated by a flexible linker is interposed between the first and second antigen binding domains. In one aspect, a human CH2 domain is placed between an ABD and a CH3 domain. In one aspect, the first and second antigen binding domains each comprise an immunoglobulin variable heavy domain and a variable light domain. In one aspect, the first and second antigen binding domains are each a scFv.

Examples include polypeptides with domain arrangements below, where the polypeptide chains can associate with one another by CH1-CK heterodimerization (linkage shown between CH1 and CK), forming interchain non-covalent bonds, and optionally further disulfide bonds) between respective complementary CH1 and CK domains.

One example is shown as follows: wherein each of the two scFv forms an antigen binding domain that binds an antigen of interest (e.g. two different antigens), and wherein the CH1 or CK constant region of one chain is a CH1 and the CH1 or CK constant region in the other chain is a CK, such that the CH1 and CK in the respective chains associate by non-covalent bonds (and optionally further disulfide bonds). Each polypeptide chain may optionally comprise one or more domains at its N- and/or C- terminus. In one aspect, a hinge domain is placed between the CH1 or CK domain and the CH2 domain.

In one aspect of any multispecific polypeptide, each CH3 domain (or fragment thereof) has the amino acid of a native human CH3 domain (or fragment thereof). Optionally, the multispecific polypeptide (and/or its Fc domain) has decreased binding to a human Fcγ receptor, e.g., compared to a full length wild type human IgG1 antibody. In one aspect, the first and second antigen binding domains are separated by an Fc domain that comprises, from N- to C- terminal, a human CH2 domain, a first human CH3 domain, a flexible amino acid linker, and a second human CH3 domain.

In one aspect of any multispecific polypeptide, the two CH3 domains associate with one another via non-covalent interactions. In one aspect, the two CH3 domains associate with one another via the CH3 dimerization interface of each CH3 domain. In one aspect, the tandem CH3 domain is incapable of further CH3-CH3 dimerization (i.e. with a further CH3 domain or polypeptide comprising such).

In one aspect, the multispecific polypeptide (and/or its Fc domain) is capable of binding to human neonatal Fc receptor (FcRn).

In one aspect, the multispecific polypeptide does not form a dimer with another Fc-derived polypeptide (e.g. does not form a homodimer with another identical polypeptide).

In one examples of bispecific polypeptides, tandem CH3 domains can be comprised in a polypeptide chain having the following domain arrangement, which polypeptide chain can remain monomeric or can associate with further polypeptide chains to form multimeric polypeptides with monomeric Fc domains:
(ABD₁) - CH2 - CH3 - linker - CH3 - (ABD₂)

Optionally, the polypeptide further comprises linking amino acids between the aforementioned domains. In one aspect, a CH1 or CK domain or fragment thereof, a hinge region or fragment thereof, and/or a linker peptide can be placed between ABD₁ and CH2. In one aspect, a CH1 or CK domain or fragment thereof, a hinge region or fragment thereof, and/or a linker peptide can be placed between CH3 and ABD₂.

Examples of multimeric antibody-derived bispecific polypeptides with monomeric Fc domains include polypeptides with domain arrangements below, where the polypeptide chains can associate with one another by CH1-CK heterodimerization (linkage shown between CH1 and CK), forming interchain disulfide bonds between respective hinge domains and/or between complementary CH1 and CK domains: or or or or or or or or

In one aspect of any aspect herein, ABD₁ and/or ABD₂ each independently comprise a scFv, an affibody, a V_{H} domain, a V_{L} domain, a single domain antibody (nanobody) such as a V-NAR domain or a V_{H}H domain. Optionally, ABD₁ and ABD₂ each comprise a scFv, an affibody, a V_{H} domain, a V_{L} domain, or a single domain antibody (nanobody) such as a V-NAR domain or a V_{H}H domain. Optionally, each ABD₁ and ABD₂ are each a human or humanized scFv, an affibody, a V_{H} domain, a V_{L} domain, a single domain antibody (nanobody) such as a V-NAR domain or a V_{H}H domain.

In one aspect of any aspect herein, the polypeptide may (or may not) further comprise additional protein domains at the C- and/or N-terminus, e.g. a further ABD, variable domain, scFv, etc.

In one aspect, the polypeptide (and/or its Fc domain) has decreased binding (i.e. via its Fc domain) to a human Fcγ receptor (e.g. CD16, CD32A, CD32B and/or CD64), e.g., compared to a full length wild type human IgG1 antibody. In one aspect, the polypeptide has decreased (e.g. partial or complete loss of) antibody dependent cytotoxicity (ADCC), complement dependent cytotoxicity (CDC), antibody dependent cellular phagocytosis (ADCP), FcR-mediated cellular activation (e.g. cytokine release through FcR cross-linking), and/or FcR-mediated platelet activation/depletion, as mediated by immune effector cells that do not express an antigen of interest bound by the antigen binding domains of the polypeptide (i.e. in the absence of cells that express an antigen of interest bound by the antigen binding domains), compared, e.g., to the same polypeptide having a wild-type Fc domain of human IgG1 isotype.

An antigen binding domain, when present, may comprise an antibody heavy chain variable domain and a light chain variable domain, optionally the first antigen binding domain comprises a scFv.

In one aspect, a first or second antigen binding domain, when present, each independently comprises an antibody heavy chain variable domain and a light chain variable domain, optionally the first antigen binding domain comprises a scFv.

In one aspect, the tandem CH3 domain is substantially incapable of forming a CH3-CH3 dimer with another Fc-derived polypeptide in solution. For example, the tandem CH3 domain does not form a dimer with another identical polypeptide, or does not form a dimer with a wild-type human IgG heavy chain.

In one aspect, a CH2 domain comprises an amino acid modification that decreases binding to a human Fcγ receptor, compared to a wild-type CH2 domain. In one aspect, the Fc polypeptide comprises N-linked glycosylation at residue N297. In one aspect, the Fc polypeptide lacks N-linked glycosylation or has modified N-linked glycosylation at residue N297. In one aspect, the Fc polypeptide comprises an N297X mutation (EU numbering), wherein X is any amino acid other than asparagine.

In one aspect of any of the aspects herein, a polypeptide is a bispecific polypeptide that comprises a first antigen binding domain that bind a cancer antigen and a second antigen binding domain that binds an antigen expressed by an immune effector cell, wherein the protein has a higher binding affinity (monovalent) for a cancer antigen (or a viral or bacterial antigen) than for an antigen expressed by an immune effector cell. Such polypeptide will provide for advantageous pharmacological properties. The polypeptide will more closely mimic (albeit with better specificity) a natural antibody that has high avidity for target antigen and lower affinity for Fc receptors. In one aspect of any of the aspects of the disclosure, the polypeptide has a Kd for binding (monovalent) to an antigen expressed by immune effector cell of less than 10⁻⁶ M, optionally less than 10⁻⁷ M, optionally less than 10⁻⁸ M, or optionally less than 10⁻⁹ M. In one aspect of any of the aspects of the disclosure, the polypeptide has a Kd for binding (monovalent) to an antigen expressed by immune effector cell of less than 10⁻⁷ M, optionally less than 10⁻⁸ M, optionally less than 10⁻⁹ M, or optionally less than 10⁻¹⁰ Mln one aspect, the polypeptide has a Kd for binding (monovalent) to a cancer, viral or bacterial antigen that is less than (i.e. has better binding affinity than) the Kd for binding (monovalent) to the antigen expressed by immune effector cell. For example, the polypeptide has a Kd for binding (monovalent) to a cancer, viral or bacterial antigen that is at least 5-fold less, optionally at least 10-fold less, than the Kd for binding (monovalent) to the antigen expressed by immune effector cell

In one aspect of any of the polypeptides herein, the multispecific polypeptide is capable of directing effector cells (e.g. a T cell, an NK cell) expressing one of first or second antigen of interest to lyse a target cell expressing the other of said first of second antigen of interest (e.g. a cancer cell).

In one aspect of any of the aspects herein, provided is a recombinant nucleic acid encoding a polypeptide chain of any of the proteins of the disclosure. In one aspect of any of the aspects herein, provided is a recombinant host cell comprising a nucleic acid encoding a polypeptide chain of any of the proteins of the disclosure, optionally wherein the host cell produces a protein of the disclosure with a yield (final productivity after purification) of at least 1, 2, 3 or 4 mg/L. Also provided is a kit or set of nucleic acids comprising a recombinant nucleic acid encoding a first polypeptide chain of the disclosure, a recombinant nucleic acid encoding a second polypeptide chain of the disclosure, and, optionally, a recombinant nucleic acid encoding a third, fourth and/or fifth polypeptide chain of the disclosure. Also provided are methods of making monomeric or multimeric proteins of the disclosure.

In one aspect, the disclosure provides methods of making a protein (e.g. any heterodimeric, trimeric, tetrameric or pentameric protein), comprising:
a) providing a first nucleic acid encoding a polypeptide chain comprising a tandem CH3 domain as described herein;
b) optionally providing a further nucleic acid encoding a further polypeptide chain described herein (e.g. a chain that associates with the chain of (a) via non-covalent and/or covalent interactions); and
c) expressing said first (and optionally further) nucleic acids in a host cell to produce a protein comprising said first (and optionally the further) polypeptide chain, respectively; and recovering a monomeric (or optionally heterodimeric, heterotrimeric, heterotetrameric or pentameric) protein. Optionally step (c) comprises loading the protein produced onto an affinity purification support, optionally an affinity exchange column, optionally a Protein-A support or column, and collecting the heterodimeric protein; and/or loading the protein produced (e.g., the protein collected following loading onto an affinity exchange or Protein A column) onto an ion exchange column; and collecting the monomeric (or optionally heterodimeric heterotrimeric, heterotetrameric or pentameric) fraction.

In one aspect of any of the proteins herein, provided is a composition comprising a plurality of tandem-CH3 domain containing proteins described herein, wherein at least 70%, 80%, 90%, 95% or 98% of the tandem-CH3 domain containing proteins have a monomeric Fc domain (do not have dimeric Fc domains upon dimerization with a second polypeptide). In one aspect, at least 70%, 80%, 90%, 95% or 98% of the tandem-CH3 domain containing proteins are monomers (i.e. single chain polypeptides). In one aspect, at least 70%, 80%, 90%, 95% or 98% of the tandem-CH3 domain containing proteins are dimers. In one aspect, at least 70%, 80%, 90%, 95% or 98% of the tandem-CH3 domain containing proteins are trimers. In one aspect, at least 70%, 80%, 90%, 95% or 98% of the tandem-CH3 domain containing proteins are tetramers.

Any of the methods can further be characterized as comprising any step described in the application, including notably in the "Detailed Description of the Invention"). The disclosure further relates to an antibody obtainable by any of present methods. The disclosure further relates to pharmaceutical or diagnostic formulations of the antibodies of the present disclosure. The disclosure further relates to methods of using antibodies in methods of treatment or diagnosis.

These and additional advantageous aspects and features of the disclosure may be further described elsewhere herein.

### BRIEF DESCRIPTION OF THE FIGURES

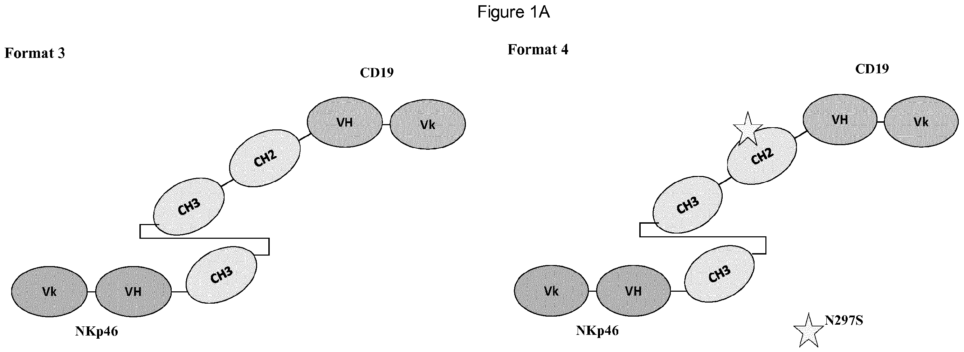

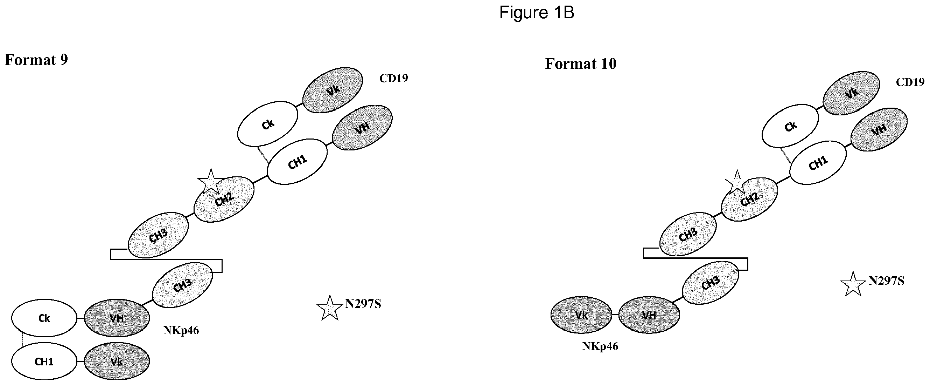

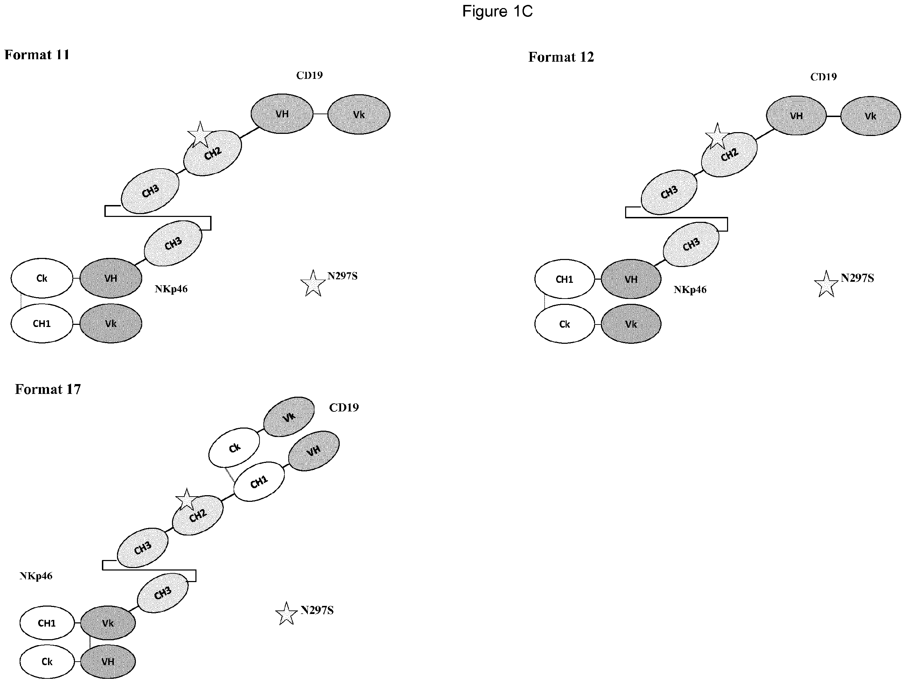

Figures 1A, 1B and 1C show examples of monomeric multispecific polypeptides comprising a monomeric Fc domain with tandem CH3 domains.

### DETAILED DESCRIPTION OF THE INVENTION

### Definitions

As used in the specification, "a" or "an" may mean one or more. As used in the claim(s), when used in conjunction with the word "comprising", the words "a" or "an" may mean one or more than one.

Where "comprising" is used, this can optionally be replaced by "consisting essentially of", or optionally by "consisting of".

As used herein, the term "antigen binding domain" refers to a domain comprising a three-dimensional structure capable of immunospecifically binding to an epitope. Thus, in one aspect, said domain can comprise a hypervariable region, optionally a VH and/or VL domain of an antibody chain, optionally at least a VH domain. In another aspect, the binding domain may comprise at least one complementarity determining region (CDR) of an antibody chain. In another aspect, the binding domain may comprise a polypeptide domain from a non-immunoglobulin scaffold.

The term "antibody" herein is used in the broadest sense and specifically includes full-length monoclonal antibodies, polyclonal antibodies, multispecific antibodies (e.g., bispecific antibodies), and antibody fragments and derivatives, so long as they exhibit the desired biological activity. Various techniques relevant to the production of antibodies are provided in, e.g., Harlow, et al., ANTIBODIES: A LABORATORY MANUAL, Cold Spring Harbor Laboratory Press, Cold Spring Harbor, N.Y., (1988). An "antibody fragment" comprises a portion of a full-length antibody, e.g. antigen-binding or variable regions thereof. Examples of antibody fragments include Fab, Fab', F(ab)₂, F(ab')₂, F(ab)₃, Fv (typically the VL and VH domains of a single arm of an antibody), single-chain Fv (scFv), dsFv, Fd fragments (typically the VH and CH1 domain), and dAb (typically a VH domain) fragments; VH, VL, VhH, and V-NAR domains; minibodies, diabodies, triabodies, tetrabodies, and kappa bodies (see, e.g., III et al., Protein Eng 1997;10: 949-57); camel IgG; IgNAR; and multispecific antibody fragments formed from antibody fragments, and one or more isolated CDRs or a functional paratope, where isolated CDRs or antigen-binding residues or polypeptides can be associated or linked together so as to form a functional antibody fragment. Various types of antibody fragments have been described or reviewed in, e.g., Holliger and Hudson, Nat Biotechnol 2005; 23, 1126-1136; WO2005040219, and published U.S. Patent Applications 20050238646 and 20020161201.

The term "antibody derivative", as used herein, comprises a full-length antibody or a fragment of an antibody, e.g. comprising at least antigen-binding or variable regions thereof, wherein one or more of the amino acids are chemically modified, e.g., by alkylation, PEGylation, acylation, ester formation or amide formation or the like. This includes, but is not limited to, PEGylated antibodies, cysteine-PEGylated antibodies, and variants thereof.

The term "hypervariable region" when used herein refers to the amino acid residues of an antibody that are responsible for antigen binding. The hypervariable region generally comprises amino acid residues from a "complementarity-determining region" or "CDR" (e.g. residues 24-34 (L1), 50-56 (L2) and 89-97 (L3) in the light-chain variable domain and 31-35 (H1), 50-65 (H2) and 95-102 (H3) in the heavy-chain variable domain; Kabat et al. 1991) and/or those residues from a "hypervariable loop" (e.g. residues 26-32 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987;196:901-917). Typically, the numbering of amino acid residues in this region is performed by the method described in Kabat et al., supra. Phrases such as "Kabat position", "variable domain residue numbering as in Kabat" and "according to Kabat" herein refer to this numbering system for heavy chain variable domains or light chain variable domains. Using the Kabat numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of CDR H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

By "framework" or "FR" residues as used herein is meant the region of an antibody variable domain exclusive of those regions defined as CDRs. Each antibody variable domain framework can be further subdivided into the contiguous regions separated by the CDRs (FR1, FR2, FR3 and FR4).

By "constant region" as defined herein is meant an antibody-derived constant region that is encoded by one of the light or heavy chain immunoglobulin constant region genes. By "constant light chain" or "light chain constant region" as used herein is meant the region of an antibody encoded by the kappa (Ckappa) or lambda (Clambda) light chains. The constant light chain typically comprises a single domain, and as defined herein refers to positions 108-214 of Ckappa, or Clambda, wherein numbering is according to the EU index (Kabat et al., 1991, Sequences of Proteins of Immunological Interest, 5th Ed., United States Public Health Service, National Institutes of Health, Bethesda). By "constant heavy chain" or "heavy chain constant region" as used herein is meant the region of an antibody encoded by the mu, delta, gamma, alpha, or epsilon genes to define the antibody's isotype as IgM, IgD, IgG, IgA, or IgE, respectively. For full length IgG antibodies, the constant heavy chain, as defined herein, refers to the N-terminus of the CH1 domain to the C-terminus of the CH3 domain, thus comprising positions 118-447, wherein numbering is according to the EU index.

By "Fab" or "Fab region" as used herein is meant the polypeptide that comprises the VH, CH1, VL, and CL immunoglobulin domains. Fab may refer to this region in isolation, or this region in the context of a polypeptide, multispecific polypeptide or ABD, or any other aspects as outlined herein.

By "single-chain Fv" or "scFv" as used herein are meant antibody fragments comprising the VH and VL domains of an antibody, wherein these domains are present in a single polypeptide chain. Generally, the Fv polypeptide further comprises a polypeptide linker between the VH and VL domains which enables the scFv to form the desired structure for antigen binding. Methods for producing scFvs are well known in the art. For a review of methods for producing scFvs see Pluckthun in The Pharmacology of Monoclonal Antibodies, vol. 113, Rosenburg and Moore eds. Springer-Verlag, New York, pp. 269-315 (1994).

By "Fv" or "Fv fragment" or "Fv region" as used herein is meant a polypeptide that comprises the VL and VH domains of a single antibody.

By "Fc" or "Fc region", as used herein is meant the polypeptide comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM, Fc may include the J chain. For IgG, Fc comprises immunoglobulin domains Cγ2 (CH2) and Cγ3 (CH3) and the hinge between Cγ1 and Cγ2. Although the boundaries of the Fc region may vary, the human IgG heavy chain Fc region is usually defined to comprise residues C226, P230 or A231 to its carboxyl-terminus, wherein the numbering is according to the EU index. Fc may refer to this region in isolation, or this region in the context of an Fc polypeptide, as described below. By "Fc polypeptide" or "Fc-derived polypeptide" as used herein is meant a polypeptide that comprises all or part of an Fc region. Fc polypeptides include but is not limited to antibodies, Fc fusions and Fc fragments.

The "hinge", "hinge region" or "hinge domain" of an IgG is generally defined as including Glu216 and terminating at Pro230 of human IgG1 according to the Kabat system but functionally, the flexible portion of the chain may be considered to include additional residues termed the upper and lower hinge regions, such as from Glu216 to Gly237 (Roux et al., 1998 J Immunol 161:4083) and the lower hinge has been referred to as residues 233 to 239 of the Fc region. As used herein, the "hinge" encompasses the upper and lower hinge regions. Hinge regions of other IgG isotypes may be aligned with the IgG1 sequence by placing the first and last cysteine residues forming inter-heavy chain S-S binds. Although boundaries may vary slightly, as numbered according to the Kabat system, the CH1 domain is adjacent to the VH domain and amino terminal to the hinge region of an immunoglobulin heavy chain molecule and includes the first (most amino terminal) constant region domain of an immunoglobulin heavy chain, e.g., from about EU positions 118-215. The Fc domain extends from amino acid 231 to amino acid 447; the CH2 domain is from about Ala231 to Lys340 or Gly341 and the CH3 from about Gly341 or Gln342 to Lys447. The residues of the IgG heavy chain constant region of the CH1 region terminate at Lys. The EU numbering system is conventionally used in the art (see generally, Kabat et al, Sequences of Protein of Immunological Interest, NIH Publication No. 91-3242, US Department of Health and Human Services (1991)).

By "variable region" as used herein is meant the region of an antibody that comprises one or more Ig domains substantially encoded by any of the VL (including Vkappa and Vlambda) and/or VH genes that make up the light chain (including kappa and lambda) and heavy chain immunoglobulin genetic loci respectively. A light or heavy chain variable region (VL and VH) consists of a "framework" or "FR" region interrupted by three hypervariable regions referred to as "complementarity determining regions" or "CDRs". The extent of the framework region and CDRs have been precisely defined, for example as in Kabat (see "Sequences of Proteins of Immunological Interest," E. Kabat et al., U.S. Department of Health and Human Services, (1983)), and as in Chothia. The framework regions of an antibody, that is the combined framework regions of the constituent light and heavy chains, serves to position and align the CDRs, which are primarily responsible for binding to an antigen.

The term "specifically binds to" means that an antibody or polypeptide can bind preferably in a competitive binding assay to the binding partner, as assessed using either recombinant forms of the proteins, epitopes therein, or native proteins present on the surface of isolated target cells. Competitive binding assays and other methods for determining specific binding are further described below and are well known in the art.

The term "affinity", as used herein, means the strength of the binding of an antibody or polypeptide to an epitope. The affinity of an antibody is given by the dissociation constant K_{D}, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant K_{A} is defined by 1/K_{D}. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One preferred and standard method well known in the art for determining the affinity of mAbs is the use of surface plasmon resonance (SPR) screening (such as by analysis with a BIAcore™ SPR analytical device).

By "amino acid modification" herein is meant an amino acid substitution, insertion, and/or deletion in a polypeptide sequence. An example of amino acid modification herein is a substitution. By "amino acid modification" herein is meant an amino acid substitution, insertion, and/or deletion in a polypeptide sequence. By "amino acid substitution" or "substitution" herein is meant the replacement of an amino acid at a given position in a protein sequence with another amino acid. For example, the substitution Y50W refers to a variant of a parent polypeptide, in which the tyrosine at position 50 is replaced with tryptophan. A "variant" of a polypeptide refers to a polypeptide having an amino acid sequence that is substantially identical to a reference polypeptide, typically a native or "parent" polypeptide. The polypeptide variant may possess one or more amino acid substitutions, deletions, and/or insertions at certain positions within the native amino acid sequence.

"Conservative" amino acid substitutions are those in which an amino acid residue is replaced with an amino acid residue having a side chain with similar physicochemical properties. Families of amino acid residues having similar side chains are known in the art, and include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

An "isolated" molecule is a molecule that is the predominant species in the composition wherein it is found with respect to the class of molecules to which it belongs (i.e., it makes up at least about 50% of the type of molecule in the composition and typically will make up at least about 70%, at least about 80%, at least about 85%, at least about 90%, at least about 95%, or more of the species of molecule, e.g., peptide, in the composition). Commonly, a composition of a polypeptide will exhibit 98%, 98%, or 99% homogeneity for polypeptides in the context of all present peptide species in the composition or at least with respect to substantially active peptide species in the context of proposed use.

In the context herein, "treatment" or "treating" refers to preventing, alleviating, managing, curing or reducing one or more symptoms or clinically relevant manifestations of a disease or disorder, unless contradicted by context. For example, "treatment" of a patient in whom no symptoms or clinically relevant manifestations of a disease or disorder have been identified is preventive or prophylactic therapy, whereas "treatment" of a patient in whom symptoms or clinically relevant manifestations of a disease or disorder have been identified generally does not constitute preventive or prophylactic therapy.

As used herein, "NK cells" refers to a sub-population of lymphocytes that is involved in non-conventional immunity. NK cells can be identified by virtue of certain characteristics and biological properties, such as the expression of specific surface antigens including CD56 and/or NKp46 for human NK cells, the absence of the alpha/beta or gamma/delta TCR complex on the cell surface, the ability to bind to and kill cells that fail to express "self" MHC/HLA antigens by the activation of specific cytolytic machinery, the ability to kill tumor cells or other diseased cells that express a ligand for NK activating receptors, and the ability to release protein molecules called cytokines that stimulate or inhibit the immune response. Any of these characteristics and activities can be used to identify NK cells, using methods well known in the art. Any subpopulation of NK cells will also be encompassed by the term NK cells. Within the context herein "active" NK cells designate biologically active NK cells, including NK cells having the capacity of lysing target cells or enhancing the immune function of other cells. For instance, an "active" NK cell can be able to kill cells that express a ligand for an activating NK receptor and/or fail to express MHC/HLA antigens recognized by a KIR on the NK cell. NK cells can be obtained by various techniques known in the art, such as isolation from blood samples, cytapheresis, tissue or cell collections, etc. Useful protocols for assays involving NK cells can be found in Natural Killer Cells Protocols (edited by Campbell KS and Colonna M). Human Press. pp. 219-238 (2000).

As used herein, "T cells" refers to a sub-population of lymphocytes that mature in the thymus, and which display, among other molecules T cell receptors on their surface. T cells can be identified by virtue of certain characteristics and biological properties, such as the expression of specific surface antigens including the TCR, CD4 or CD8, the ability of certain T cells to kill tumor or infected cells, the ability of certain T cells to activate other cells of the immune system, and the ability to release protein molecules called cytokines that stimulate or inhibit the immune response. Any of these characteristics and activities can be used to identify T cells, using methods well known in the art. Within the context herein, "active" or "activated" T cells designate biologically active T cells, more particularly T cells having the capacity of cytolysis or of stimulating an immune response by, e.g., secreting cytokines. Active cells can be detected in any of a number of well-known methods, including functional assays and expression-based assays such as the expression of cytokines such as TNF-alpha.

### Producing polypeptides

The disclosure herein relates to a tandem CH3 domain and to polypeptides that comprise a tandem CH3 domain. The tandem CH3 domain can be configured within a single polypeptide chain so that the two CH3 domains interact by forming CH3-CH3 dimers. Such CH3 domains will then not be available for further dimerization with other CH3-domain containing polypeptides, notably Fc-domain-containing polypeptides. Any amino acid sequence, polypeptide domain or polypeptide of interest, e.g. a "parent", "starting" or "nonvariant" polypeptide, can be used as starting point or as a source of amino acid sequence to be incorporated into a tandem CH3 domain-containing polypeptide using techniques available in the art for generating polypeptides. The tandem CH3 domain-containing polypeptide can for example comprise a human Fc region comprising a CH2 domain linked to a CH3 domain, wherein the CH3 domains is one of the tandem CH3 domains.

The parent polypeptide may be any polypeptide comprising an Fc region, for example an Fc fusion protein comprising an Fc domain (e.g. a dimeric Fc domain). Examples herein comprise an Fc domain and an antigen binding domain from an antibody (e.g. a variable domain).

The Fc domains can be useful for extending the half-life of any protein of interest, or otherwise to provide a desired functionality conferred by the Fc domain, e.g. recognition by a secondary reagent, binding to a solid support for purification, etc. Thus, the Fc domain molecules can include any heterologous polypeptide. The fused partner can be any other proteinaceous molecule of interest, such as an enzyme, a detectable molecule, a ligand that activates upon interaction with a cell-surface receptor, a peptidic antigen (Ag) against a challenging pathogen or a 'bait' protein to identify binding partners assembled in a protein microarray.

Examples of proteins include labels, receptors, growth factors, ligands, enzymes, erythropoietin, chemokines, antigen binding domain from a non-immunoglobulin scaffold, therapeutic proteins and hormones. In some non-limiting examples, the heterologous protein is a human interferon, erythropoietin (EPO), soluble tumor necrosis factor receptor, CTLA-4, soluble interleukin (IL)-4 receptor, or Factor IX. Examples of Fc-domain containing molecules that can be used as monomeric or multimeric proteins with monomeric Fc domain and modified by incorporating a tandem CH3 domain include: CTLA-4 fused to the Fc of human IgG1 (Nulojix (belatacept), for the treatment of organ rejection); VEGFR1/VEGFR2 fused to the Fc of human IgG1 (Eylea (aflibercept), for the treatment of age related macular degeneration); IL-1R fused to the Fc of human IgG1 (Arcalyst (rilonacept), for the treatment of cryopyrin-associated periodic syndromes); Thrombopoietin-binding peptide fused to the Fc of human IgG1 (NPlate (romiplostim), for the treatment of thrombocytopenia in chronic immune thrombocytopenic purpura patients); Mutated CTLA-4 fused to the Fc of human IgG1 (Orencia (abatacept), for the treatment of rheumatoid arthritis); LFA-3 fused to the Fc of human IgG1 (Amevive (alefacept), for the treatment of psoriasis and transplant rejection); and TNFR fused to the Fc of human IgG1 (Enbrel (etanercept), for the treatment of rheumatoid arthritis).

Exemplary methods for generating Fc-polypeptides that are antibodies are described in more detail in the following sections. The antigen binding domains used in the polypeptides described herein can be readily derived a variety of immunoglobulin or non-immunoglobulin scaffolds, for example affibodies based on the Z-domain of staphylococcal protein A, engineered Kunitz domains, monobodies or adnectins based on the 10th extracellular domain of human fibronectin III, anticalins derived from lipocalins, DARPins (desiged ankyrin repeat domains, multimerized LDLR-A module, avimers or cysteine-rich knottin peptides. See, e.g., Gebauer and Skerra (2009) Current Opinion in Chemical Biology 13:245-255.

Antigen binding domains are commonly derived from antibodies (immunoglobulin chains), for example in the form of associated VL and VH domains found on two polypeptide chains, or single chain antigen binding domains such as scFv, a V_{H} domain, a V_{L} domain, a dAb, a V-NAR domain or a V_{H}H domain. An antigen binding domain (e.g., ABD₁ and ABD₂) can also be readily derived from antibodies as a Fab.

Typically, antibodies are initially obtained by immunization of a non-human animal, e.g., a mouse, with an immunogen comprising a polypeptide, or a fragment or derivative thereof, typically an immunogenic fragment, for which it is desired to obtain antibodies (e.g. a human polypeptide). The step of immunizing a non-human mammal with an antigen may be carried out in any manner well known in the art for stimulating the production of antibodies in a mouse (see, for example, E. Harlow and D. Lane, Antibodies: A Laboratory Manual., Cold Spring Harbor Laboratory Press, Cold Spring Harbor, NY (1988)).

Human antibodies may also be produced by using, for immunization, transgenic animals that have been engineered to express a human antibody repertoire (Jakobovitz et Nature 362 (1993) 255), or by selection of antibody repertoires using phage display methods. For example, a XenoMouse (Abgenix, Fremont, CA) can be used for immunization. A XenoMouse is a murine host that has had its immunoglobulin genes replaced by functional human immunoglobulin genes. Thus, antibodies produced by this mouse or in hybridomas made from the B cells of this mouse, are already humanized. The XenoMouse is described in United States Patent No. 6,162,963.

Antibodies may also be produced by selection of combinatorial libraries of immunoglobulins, as disclosed for instance in (Ward et al. Nature, 341 (1989) p. 544). Phage display technology (McCafferty et al (1990) Nature 348:552-553) can be used to produce antibodies from immunoglobulin variable (V) domain gene repertoires from unimmunized donors. See, e.g., Griffith et al (1993) EMBO J. 12:725- 734; US 5565332; US 5573905; US 5567610; US 5229275). When combinatorial libraries comprise variable (V) domain gene repertoires of human origin, selection from combinatorial libraries will yield human antibodies.

Additionally, a wide range of antibodies are available in the scientific and patent literature, including DNA and/or amino acid sequences, or from commercial suppliers. Antibodies will typically be directed to a pre-determined antigen. Examples of antibodies include antibodies that recognize an antigen expressed by a target cell that is to be eliminated, for example a proliferating cell or a cell contributing to pathology. Examples include antibodies that recognize tumor antigens, microbial (e.g. bacterial) antigens or viral antigens.

Antigen binding domains (ABDs) can be selected based on the desired cellular target, and may include for example cancer antigens, bacterial or viral antigens, etc. As used herein, the term "bacterial antigen" includes, but is not limited to, intact, attenuated or killed bacteria, any structural or functional bacterial protein or carbohydrate, or any peptide portion of a bacterial protein of sufficient length (typically about 8 amino acids or longer) to be antigenic. Examples include gram-positive bacterial antigens and gram-negative bacterial antigens. In some aspects the bacterial antigen is derived from a bacterium selected from the group consisting of Helicobacter species, in particular Helicobacter pyloris; Borelia species, in particular Borelia burgdorferi; Legionella species, in particular Legionella pneumophilia; Mycobacteria s species, in particular M. tuberculosis, M. avium, M. intracellulare, M. kansasii, M. gordonae; Staphylococcus species, in particular Staphylococcus aureus; Neisseria species, in particular N. gonorrhoeae, N. meningitidis; Listeria species, in particular Listeria monocytogenes; Streptococcus species, in particular S. pyogenes, S. agalactiae; S. faecalis; S. bovis, S. pneumonias; anaerobic Streptococcus species; pathogenic Campylobacter species; Enterococcus species; Haemophilus species, in particular Haemophilus influenzue; Bacillus species, in particular Bacillus anthracis; Corynebacterium species, in particular Corynebacterium diphtheriae; Erysipelothrix species, in particular Erysipelothrix rhusiopathiae; Clostridium species, in particular C. perfringens, C. tetani; Enterobacter species, in particular Enterobacter aerogenes, Klebsiella species, in particular Klebsiella 1S pneumoniae, Pasturella species, in particular Pasturella multocida, Bacteroides species; Fusobacterium species, in particular Fusobacterium nucleatum; Streptobacillus species, in particular Streptobacillus moniliformis; Treponema species, in particular Treponema pertenue; Leptospira; pathogenic Escherichia species; and Actinomyces species, in particular Actinomyces israelli.

As used herein, the term "viral antigen" includes, but is not limited to, intact, attenuated or killed whole virus, any structural or functional viral protein, or any peptide portion of a viral protein of sufficient length (typically about 8 amino acids or longer) to be antigenic. Sources of a viral antigen include, but are not limited to viruses from the families: Retroviridae (e.g., human immunodeficiency viruses, such as HIV-1 (also referred to as HTLV-III, LAV or HTLV-III/LAV, or HIV-III; and other isolates, such as HIV-LP; Picornaviridae (e.g., polio viruses, hepatitis A virus; enteroviruses, human Coxsackie viruses, rhinoviruses, echoviruses); Calciviridae (e.g., strains that cause gastroenteritis); Togaviridae (e.g., equine encephalitis viruses, rubella viruses); Flaviviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g., influenza viruses); Bunyaviridae (e.g., Hantaan viruses, bunya viruses, phleboviruses and Nairo viruses); Arenaviridae (hemorrhagic fever viruses); Reoviridae (e.g., reoviruses, orbiviruses and rotaviruses); Bornaviridae; Hepadnaviridae (Hepatitis B virus); Parvoviridae (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus; Poxyiridae (variola viruses, vaccinia viruses, pox viruses); and Iridoviridae (e.g., African swine fever virus); and unclassified viruses (e.g., the agent of delta hepatitis (thought to be a defective satellite of hepatitis B virus), Hepatitis C; Norwalk and related viruses, and astroviruses). Alternatively, a viral antigen may be produced recombinantly.

As used herein, the terms "cancer antigen" and "tumor antigen" are used interchangeably and refer to antigens (other than NKp46 or CD16) that are differentially expressed by cancer cells or are expressed by non-tumoral cells (e.g. immune cells) having a pro-tumoral effect (e.g. an immunosuppressive effect), and can thereby be exploited in order to target cancer cells. Cancer antigens are antigens which can potentially stimulate apparently tumor-specific immune responses. Some of these antigens are encoded, although not necessarily expressed, or expressed at lower levels or less frequently, by normal cells. These antigens can be characterized as those which are normally silent (i.e., not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses. Still other cancer antigens can be expressed on immune cells capable of mediating a pro-tumoral effect, e.g. a monocyte or a macrophage, optionally a suppressor T cell, regulatory T cell, or myeloid-derived suppressor cell.

The cancer antigens are usually normal cell surface antigens which are either over-expressed or expressed at abnormal times, or are expressed by a targeted population of cells. Ideally the target antigen is expressed only on proliferative cells (e.g., tumor cells) or pro-tumoral cells (e.g. immune cells having an immunosuppressive effect), however this is rarely observed in practice. As a result, target antigens are in many cases selected on the basis of differential expression between proliferative/disease tissue and healthy tissue. Example of cancer antigens include: Receptor Tyrosine Kinase-like Orphan Receptor 1 (ROR1), Cripto, CD4, CD20, CD30, CD19, CD38, CD47, Glycoprotein NMB, CanAg, Her2 (ErbB2/Neu), a Siglec family member, for example CD22 (Siglec2) or CD33 (Siglec3), CD79, CD138, CD171, PSCA, L1-CAM, PSMA (prostate specific membrane antigen), BCMA, CD52, CD56, CD80, CD70, E-selectin, EphB2, Melanotransferin, Mud 6 and TMEFF2. Examples of cancer antigens also include Immunoglobulin superfamily (IgSF) such as cytokine receptors, Killer-Ig Like Receptor, CD28 family proteins, for example, Killer-Ig Like Receptor 3DL2 (KIR3DL2), B7-H3, B7-H4, B7-H6, PD-L1, IL-6 receptor. Examples also include MAGE, MART-1/Melan-A, gp100, major histocompatibility complex class I-related chain A and B polypeptides (MICA and MICB), adenosine deaminase-binding protein (ADAbp), cyclophilin b, colorectal associated antigen (CRC)-C017-1A/GA733, protein tyrosine kinase 7(PTK7), receptor protein tyrosine kinase 3 (TYRO-3), nectins (e.g. nectin-4), major histocompatibility complex class I-related chain A and B polypeptides (MICA and MICB), proteins of the UL16-binding protein (ULBP) family, proteins of the retinoic acid early transcript-1 (RAET1) family, carcinoembryonic antigen (CEA) and its immunogenic epitopes CAP-1 and CAP-2, etv6, aml1, prostate specific antigen (PSA), T-cell receptor/CD3-zeta chain, MAGE-family of tumor antigens, GAGE-family of tumor antigens, anti-Mullerian hormone Type II receptor, delta-like ligand 4 (DLL4), DR5, ROR1 (also known as Receptor Tyrosine Kinase-Like Orphan Receptor 1 or NTRKR1 (EC 2.7.10.1), BAGE, RAGE, LAGE-1, NAG, GnT-V, MUM-1, CDK4, MUC family, VEGF, VEGF receptors, Angiopoietin-2, PDGF, TGF-alpha, EGF, EGF receptor, members of the human EGF-like receptor family, e.g., HER-2/neu, HER-3, HER-4 or a heterodimeric receptor comprised of at least one HER subunit, gastrin releasing peptide receptor antigen, Muc-1, CA125, integrin receptors, αvβ3 integrins, α5β1 integrins, αllbβ3-integrins, PDGF beta receptor, SVE-cadherin, IL-8 receptor, hCG, IL-6 receptor, CSF1R (tumor-associated monocytes and macrophages), α-fetoprotein, E-cadherin, α-catenin, β-catenin and γ-catenin, p120ctn, PRAME, NY-ESO-1, cdc27, adenomatous polyposis coli protein (APC), fodrin, Connexin 37, Ig-idiotype, p15, gp75, GM2 and GD2 gangliosides, viral products such as human papillomavirus proteins, imp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2, although this is not intended to be exhaustive. In one aspect, the antigen of interest is an antigen (e.g. any one of the antigens listed above) capable of undergoing intracellular internalization, for example when bound by an conventional human IgG1 antibody, either in the presence of absence of Fcγ receptor cells.

In one aspect, the antigen target is a polypeptide present on an immune cell capable of mediating a pro-tumoral effect, e.g. a monocyte or a macrophage, optionally a suppressor T cell, regulatory T cell, or myeloid-derived suppressor cell.

In exemplary aspects of a multispecific molecule comprising an ABD₁ and ABD₂, one of such ABD may be bind an antigen expressed by a target cell that is to be eliminated (e.g., a tumor antigen, microbial (e.g. bacterial) antigen, viral antigen, or antigen expressed on an immune cell that is contributing to inflammatory or autoimmune disease, and the other of ABD₁ or ABD₂ will bind to an antigen expressed on an immune cell, for example an immune effector cell, e.g. a cell surface receptor of an effector cells such as a T or NK cell. Examples of antigens expressed on immune cells, optionally immune effector cells, include antigens expressed on a member of the human lymphoid cell lineage, e.g. a human T cell, a human B cell or a human natural killer (NK) cell, a human monocyte, a human neutrophilic granulocyte or a human dendritic cell. Advantageously, such cells will have either a cytotoxic or an apoptotic effect on a target cell that is to be eliminated (e.g., that expresses a tumor antigen, microbial antigen, viral antigen, or antigen expressed on an immune cell that is contributing to inflammatory or autoimmune disease). Especially advantageously, the human lymphoid cell is a cytotoxic T cell or NK cell which, when activated, exerts a cytotoxic effect on the target cell. According to this aspect, then, the cytotoxic activity of the human effector cells are recruited. According to another aspect, the human effector cell is a member of the human myeloid lineage.

An ABD of a multispecific polypeptide may bind to an antigen expressed on an immune cell such as an NK and/or T cell receptor, e.g. any molecule on the surface of NK cells or T cells, respectively, that can serve to direct the NK or T cell to the intended target cell to be eliminated. Examples include, e.g., members of the immunoglobulin superfamily, members of the killer-cell immunoglobulin-like receptor (KIR) family, the leukocyte immunoglobulin-like receptors (LILR) family, or the lectin family or the NK cell lectin-like receptor family. Activity can be measured for example by bringing target cells and effector cells into contact in presence of the multispecific polypeptide.

Optionally the immune cell receptor is an activating receptor, e.g. an activating NK cell or T cell receptor. As used herein, the terms "activating NK cell receptor" and "activating T cell receptor" refers to any molecule on the surface of NK cells or T cells, respectively, that, when stimulated, causes a measurable increase in any property or activity known in the art as associated with NK cell or T cell activity, respectively, such as cytokine (for example IFN-y or TNF-α) production, increases in intracellular free calcium levels, the ability to lyse target cells in a redirected killing assay as described, e.g. elsewhere in the present specification, or the ability to stimulate NK cell or T cell proliferation, respectively. The term "activating NK receptor" includes activating or co-activating receptors and includes but is not limited to such as forms of KIR proteins (for example KIR2DS receptors, KIR2DS2, KIR2DS4), NKG2 receptors, NKp30, NKp46, IL-2R, IL-12R, IL-15R, IL-18R and IL-21R. One important family of activating receptors is the natural cytotoxicity receptors (NCRs) which include NKp30, NKp44 and NKp46.

Activation of cytotoxic T cells may for example occur via binding of the CD3 antigen as effector antigen on the surface of the cytotoxic T cell by a multispecific (e.g. bispecific) polypeptide of this aspect. The human CD3 antigen is present on both helper T cells and cytotoxic T cells. Human CD3 denotes an antigen which is expressed on T cells as part of the multimolecular T cell complex and which comprises three different chains: CD3-epsilon, CD3-delta and CD3-gamma.

Exemplary lymphoid cell-associated effector antigens bound by a multispecific polypeptide are the human CD8 antigen, the human CD16 antigen, the human NKG2D antigen, the human NKp46 antigen, the human NKp30 antigen, the human CD2 antigen, the human CD28 antigen or the human CD25 antigen. The human CD8 antigen, expressed on effector T cells, can be used to redirect such T cells toward a target cell of interest, without for example affecting CD4 T cells (that could be associated with unwanted cytokine production and associated toxicity) or TReg cells. The human NKp30 and particularly the NKp46 antigen are expressed primarily on NK cells and thereby permit the selective recruitment of NK cell effector activity. In one aspect, the antigen expressed on an immune or immune effector cell is selected from a FcyRI (CD64), FcyRIIA (CD32), and an FcγRIII (CD16) polypeptide.

The ABD(s) which are incorporated into the Fc polypeptides can be tested for any desired activity prior to inclusion in a polypeptide. Once appropriate antigen binding domains having desired specificity and/or activity are identified, DNA encoding each of the or ABD can be placed, in suitable arrangements, in an appropriate expression vector(s), together with DNA encoding any elements such as CH2 and CH3 domains, linkers and any other optional elements (e.g. DNA encoding a linker or hinge region) for transfection into an appropriate host. The host is then used for the recombinant production of the Fc polypeptide.

An ABD derived from an antibody will generally comprise at minimum a hypervariable region sufficient to confer binding activity. It will be appreciated that an ABD may comprise or be fused to other amino acids or functional domains as may be desired, including but not limited to linker elements (e.g. linker peptides, CH1, Cκ or Cλ domains, hinges, or fragments thereof, each of which can be placed between an ABD and a CH2 or CH3 domain, or between other domains as needed). In one example an ABD comprises an scFv, a V_{H} domain and a V_{L} domain, or a single domain antibody (nanobody or dAb) such as a V-NAR domain or a V_{H}H domain. Exemplary antibody formats are further described herein and an ABD can be selected based on the desired format.

In any aspect, an antigen binding domain can be obtained from a humanized antibody in which residues from a complementary-determining region (CDR) of a human antibody are replaced by residues from a CDR of the original antibody (the parent or donor antibody, e.g. a murine or rat antibody) while maintaining the desired specificity, affinity, and capacity of the original antibody. The CDRs of the parent antibody, some or all of which are encoded by nucleic acids originating in a non-human organism, are grafted in whole or in part into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536. An antigen binding domain can thus have non-human hypervariable regions or CDRs and human frameworks region sequences (optionally with backmutations).

Protein domains, e.g. ABDs or ECDs, will be arranged in an expression vector so as to produce the Fc-polypeptides having the desired domains operably linked to one another. The Fc-polypeptide can then be produced in an appropriate host cell or by any suitable synthetic process. The host cell may be of mammalian origin or may be selected from COS-1, COS-7, HEK293, BHK21, CHO, BSC-1, Hep G2, 653, SP2/0, 293, HeLa, myeloma, lymphoma, yeast, insect or plant cells, or any derivative, immortalized or transformed cell thereof. Alternatively, the host cell may be selected from a species or organism incapable of generating mammalian glycosylation on antibodies, e.g. a prokaryotic cell or organism, such as natural or engineered E. coli spp., Klebsiella spp., or Pseudomonas spp.

Fc-derived polypeptide chains can be constructed that comprise: (a) a protein domain of interest (e.g., an ECD of a cell surface receptor, a VH and/or VL, an antigen binding domain (ABD₁) that binds to a first antigen of interest), (b) an Fc domain comprising a CH2 domain (or fragment thereof) and a tandem CH3 domain comprising a first CH3 domain and a second CH3 domain separated from the first CH3 domain by an intervening sequences of amino acid residues (e.g. a flexible peptide linker). Optionally the CH2 domain and the domain of interest are separated by a linker, a CH1 or CK domain, and/or a hinge region. Optionally the Fc domain (i) does not undergo CH3-CH3 dimerization with another Fc-derived polypeptide, (ii) is capable of binding to human FcRn and/or (iii) has decreased binding to a human Fcγ receptor compared to a wild type human IgG1 Fc domain. Examples include polypeptides having a domain arrangement of one of the following:
(ECD) - CH2 - CH3 - linker - CH3,
(ECD) - CH1 - CH2 - CH3 - linker - CH3,
(VH or VL) - CH2 - CH3 - linker - CH3,
(VH or VL) - CH1 - CH2 - CH3 - linker - CH3,
(ABD₁) - CH1 - CH2 - CH3 - linker - CH3, or
(ABD₁) - CH2 - CH3 - linker - CH3.

Multispecific, e.g. bispecific, Fc-derived polypeptide chains can be constructed that comprise: (a) a first protein domain of interest (e.g., an ECD of a cell surface receptor, a VH and/or a VL, an antigen binding domain (ABD₁) that binds to a first antigen of interest); (b) an Fc domain comprising a CH2 domain (of fragment thereof) and a tandem CH3 domain comprising a first CH3 domain and a second CH3 domain separated from the first CH3 domain by an intervening amino acid sequence (e.g. a flexible peptide linker), and (c) a second protein domain of interest (e.g., an ECD of a cell surface receptor, a VH and/or a VL, an antigen binding domain (ABD₂) that binds to a second antigen of interest). Each of the first and second protein domain of interest can independently be any desired amino acid sequence, e.g. an antigen binding domain, a functional peptide such as a detectable tag, an enzymatic recognition tag or a purification tag, a receptor polypeptide, an enzyme, a cytokine, etc. Examples of a polypeptide having a domain arrangement are as follows:
(domain of interest₁) - CH2 - CH3 - linker - CH3 - (domain of interest₂),
(domain of interest₁) - CH1 - CH2 - CH3 - linker - CH3 - (domain of interest₂),
(ABD₁) - CH2 - CH3 - linker - CH3 - (ABD₂),
(ABD₁) - CH1 - CH2 - CH3 - linker - CH3 - (ABD₂),
(ECD) - CH2 - CH3 - linker - CH3 - (ABD),
(ECD) - CH1 - CH2 - CH3 - linker - CH3 - (ABD),
(ABD) - CH2 - CH3 - linker - CH3 - (ECD), or
(ABD) - CH1 - CH2 - CH3 - linker - CH3 - (ECD).

Optionally a protein domain of interest is fused to the adjacent CH2 domain via a linker peptide, a CH1 or CK domain, and/or a hinge region. Optionally the CH3 domain is fused to the adjacent CH3 domain via a linker peptide. Optionally the Fc domain (i) is not capable of CH3-CH3 dimerization with another Fc-derived polypeptide, (ii) is capable of binding to human FcRn and/or (iii) has decreased binding to a human Fcγ receptor compared to a wild type human IgG1 Fc domain.

The tandem CH3 domain-containing portions of the above examples can comprise for example a domain arrangement as follows (from N-terminus to C-terminus): CH2 - CH3 - peptide linker - CH3 -.

The Fc domain or the Fc polypeptides can thus optionally be described as being or comprising a native human Fc domain comprising a CH2 domain and a CH3 domain, where the CH3 domain is fused at its free terminus to a peptide linker, which is in turn fused at its free terminus to a second CH3 domain.

Various domain arrangements can be envisaged. For example a tandem CH3 domain-containing Fc portion can be fused to the C-terminus of a tandem ABD or scFv. Non-limiting examples include polypeptide having any of the following domain arrangements (from N-terminus to C-terminus):
(ABD₁) - (ABD₂) - CH2 - CH3 - linker - CH3,
(ABD₁) - (ABD₂) - CH1 - CH2 - CH3 - linker - CH3, or
(scFv₁) - (scFv₂) - CH2 - CH3 - linker - CH3.

Other examples using scFvs include proteins having the following domain arrangement (from N-terminus to C-terminus):
(scFv₁) - CH2 - CH3 - linker - CH3 - (scFv₂) or
(scFv₁) - CH1 - CH2 - CH3 - linker - CH3 - (scFv₂).

Further examples of multispecific proteins include multimeric proteins, including proteins and domain arrangements having monomeric Fc domain shown in PCT application no. WO2015/197593. For example, a heterodimer can for example have the configuration as follows (see also Examples of such proteins shown as formats 11 and 12 shown in Figure 1C): 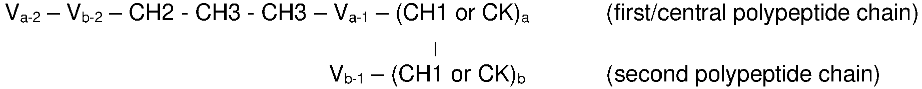 wherein one of Vₐ₋₁ of the first polypeptide chain and V_{b-1} of the second polypeptide chain is a light chain variable domain and the other is a heavy chain variable domain, and wherein one of Vₐ₋₂ and V_{b-2} is a light chain variable domain and the other is a heavy chain variable domain.

The resulting heterodimer can in another example have the configuration as follows (see also Examples of such proteins shown as format 10 shown in Figure 1B): 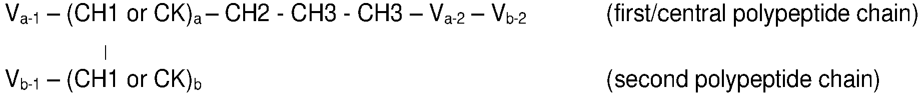 wherein one of Vₐ₋₁ of the first polypeptide chain and V_{b-1} of the second polypeptide chain is a light chain variable domain and the other is a heavy chain variable domain, and wherein one of Vₐ₋₂ and V_{b-2} is a light chain variable domain and the other is a heavy chain variable domain.

Further examples include trimers. An exemplary trimer can have the configuration as follows (also shown as format 17 in Figure 1C):

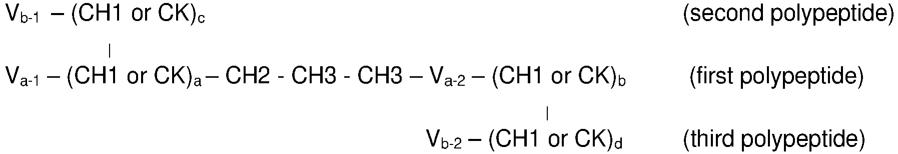

In any Fc polypeptide, a CH1 or CK (or Cλ) domain may be present between a protein or domain of interest, e.g., ABD, ECD, and a CH2 domain, or the CH1 or CK (or Cλ) domain may be absent between the domain of interest and a CH2 domain. In one aspect, a peptide linker is present between a protein or domain of interest and a CH2 domain. In one aspect, a hinge region or fragment thereof is present between a CH1 and a CH2 domain, for example a modified hinge which lacks one or both of the cysteines involved in interchain bridges in native antibody heavy chains. The first and second ABDs can be linked together by a linker of sufficient length to enable the ABDs to fold in such a way as to permit binding to the respective antigen for which the ABD is intended to bind. Suitable peptide linkers for use in linking ABD₁ to ABD₂, or for use in linking an ABD to a CH2 or CH3 are known in the art, see, e.g. WO2007/073499. Examples of linker sequences include (G₄S)ₓ wherein x is an integer (e.g. 1, 2, 3, 4, or more). The Fc polypeptide can thus for example have the structure (ABD₁ - peptide linker - ABD₂ - peptide linker - CH2 - CH3 - peptide linker - CH3). For example, the polypeptide may comprise, as a fusion product, the structure (scFv₁ - peptide linker - scFv₂-peptide linker - CH2 - CH3), wherein each element is fused to the following element. Another example of an Fc polypeptide has the structure: (ABD₁ - peptide linker - CH2 - CH3 - peptide linker - CH3 -peptide linker - ABD₂).

The arrangements for purposes of illustration are shown from N-terminus (on the left) to C-terminus. Domains can be referred to as fused to one another (e.g. a domain can be said to be fused to the C-terminus of the domain on its left, and/or a domain can be said to be fused to the N-terminus of the domain on its right). Domains can be fused to one another directly or via intervening amino acid sequences.

As shown in certain examples, a multispecific antibody can be prepared in which the two antigen binding domains are positioned on opposite termini of an Fc domain. This protein can provide for conformations that can provide better binding to target antigens on different cells. This configuration also permits a wider range of antibody variable regions to be used; some antibody binding domains that do not remain functional in tandem scFv format will remain functional in single scFv form. Thus, in one aspect the polypeptide can comprise: (a) a first antigen binding domain (ABD₁) that binds to a first antigen of interest, (b) a tandem CH3 domain-containing portion comprising at least a portion of a human Fc domain comprising a first CH3 domain, and a second CH3 domain separated from the first CH3 domain by an intervening amino acid sequence, wherein the tandem CH3 domain-containing portion is fused (optionally via intervening sequences of amino acid residues) to the C-terminus of the first antigen binding domain, and (c) a second antigen binding domain (ABD₂) that binds a second antigen of interest is fused (optionally via intervening amino acid sequences) to the C-terminus of the tandem CH3 domain-containing portion.

In one aspect of any aspect, the first antigen binding domain and/or the second antigen binding domain comprise a scFv, optionally where the scFv comprises human framework amino acid sequences.

An exemplary CH3 domain for use within a tandem CH3 domain can comprise an amino acid sequence of SEQ ID NO : 1, or a sequence at least 70%, 80%, 90%, 95% or 98% identical thereto:

An exemplary tandem CH3 with a flexible peptide linker (underlined) is shown below. An exemplary tandem CH3 domain can thus comprise an amino acid sequence of SEQ ID NO : 2, or a sequence at least 70%, 80%, 90%, 95% or 98% identical thereto:

In one aspect, a peptide linker used to link an ABD (e.g. an scFv, a VH or VL domain) to a CH2 or CH3 comprises a CH1 domain or a fragment of a CH1 domain. For example, a CH1 domain, or an N-terminal amino acid sequence of a CH1 domain can be fused to an ABD (e.g. an scFv, a VH or VL domain, etc.) in order to mimic as closely as possible the natural structure of an antibody. In one aspect, the linker comprises a N-terminal CH1 amino acid sequence of between 2-4 residues, between 2-4 residues, between 2-6 residues, between 2-8 residues, between 2-10 residues, between 2-12 residues, between 2-14 residues, between 2-16 residues, between 2-18 residues, between 2-20 residues, between 2-22 residues, between 2-24 residues, between 2-26 residues, between 2-28 residues, between 2-30 residues, between 10-20 residues, between 10-22 residues, between 10-24 residues, between 2-26 residues, between 10-28 residues, between 12-20 residues, between 15-30 residues, between 15-20 residues or between 10-30 residues. In one aspect linker comprises or consists of the amino acid sequence RTVA.

When an ABD is an scFv, the VH domain and VL domains (VL or VH domains or fragments thereof that retain binding specificity) that form a scFv are linked together by a linker of sufficient length to enable the ABD to fold in such a way as to permit binding to the antigen for which the ABD is intended to bind. Examples of linkers include, for example, linkers comprising glycine and serine residues, e.g., the amino acid sequence GEGTSTGS(G₂S)₂GGAD. In one specific aspect, the VH domain and VL domains of an scFv are linked together by the amino acid sequence (G₄S)₃.

Peptide linkers used to link the two CH3 domains to one another can be any amino acid sequence of sufficient length to enable the two CH3 domains to fold in such a way as to permit them to associate by non-covalent interactions, notably to undergo CH3-CH3 dimerization. Examples of linkers include, for example, linkers comprising glycine and serine residues, e.g., the amino acid sequence (G₄S)ₓ, wherein x is between 1 and 10, optionally between 2 and 6, e.g. (G₄S)₃.

Any of the peptide linkers used to link the two CH3 domains to one another may comprise a length of at least 5 residues, optionally at least 10 residues, at least 15 residues, at least 20 residues, at least 25 residues, at least 30 residues or more. In other aspects, the linkers comprises a length of between 5-10 residues, between 4-12 residues, between 5-14 residues, between 5-16 residues, between 5-18 residues, between 5-20 residues, between 5-22 residues, between 5-24 residues, between 5-26 residues, between 5-28 residues, between 5-30 residues, between 10-20 residues, between 10-22 residues, between 10-24 residues, between 2-26 residues, between 10-28 residues, between 12-20 residues, between 15-30 residues, between 15-20 residues or between 10-30 residues.

In one aspect, the hinge region will be a fragment of a hinge region (e.g. a truncated hinge region without cysteine residues) or may comprise one or amino acid modifications to remove (e.g. substitute by another amino acid, or delete) a cysteine residue, optionally both cysteine residues in a hinge region. Removing cysteines can be useful to prevent formation of disulfide bridges in a monomeric polypeptide.

Constant region domains can be derived from any suitable antibody. Of particular interest are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat. By "hinge" or "hinge region" or "antibody hinge region" is meant the flexible polypeptide comprising the amino acids between the first and second constant domains of an antibody. Structurally, the IgG CH1 domain ends at EU position 220, and the IgG CH2 domain begins at residue EU position 237. References to amino acid residue within constant region domains found within the polypeptides shall be, unless otherwise indicated or as otherwise dictated by context, with reference to Kabat, in the context of an IgG antibody.

CH3 domains that can serve in the present antibodies can be derived from any suitable antibody. Such CH3 domains can serve as the basis for a modified CH3 domain. Optionally the CH3 domain is of human origin. In certain aspects herein, the CH3 domains can be native human CH3 domains, while in other aspects the CH domains can comprise one or more amino acid modifications (e.g. amino acid substitutions). The modifications will generally avoid disrupting the CH3 dimerization interface, such that the tandem CH3 domains will retain the ability to associate with one another. Optionally, the CH3 domain modifications will additionally not interfere with the ability of the Fc-derived polypeptide to bind to neonatal Fc receptor (FcRn), e.g. human FcRn.

CH2 domains can be readily obtained from any suitable antibody. Optionally the CH2 domain is of human origin. A CH2 may or may not be linked (e.g. at its N-terminus) to a hinge of linker amino acid sequence. In one aspect, a CH2 domain is a naturally occurring human CH2 domain of IgG1, 2, 4 or 4 subtype. In one aspect, a CH2 domain is a fragment of a CH2 domain (e.g. at least 10, 20, 30, 40 or 50 amino acids).

In one aspect, a CH2 domain, when present in a polypeptide described herein, will retain binding to a neonatal Fc receptor (FcRn), particularly human FcRn.

In one aspect, a CH2 domain, when present in a polypeptide described herein, and the polypeptides described herein, will confer decreased or lack of binding to a Fcγ receptor, notably FcγRIIIA (CD16). Polypeptides that comprise a CH2 domain that do not bind CD16 will not be capable of activating or mediating ADCC by cells (e.g. NK cells, T cells) that do not express the effector cell antigen of interest (e.g. NKp46, CD3, etc.).

In one aspect, the polypeptides described herein and their Fc domain(s) and/or a CH2 domain thereof, will have decreased or will substantially lack antibody dependent cytotoxicity (ADCC), complement dependent cytotoxicity (CDC), antibody dependent cellular phagocytosis (ADCP), FcR-mediated cellular activation (e.g. cytokine release through FcR cross-linking), and/or FcR-mediated platelet activation/depletion.

In one aspect, a CH2 domain in a polypeptide will have substantial loss of binding to activating Fcγ receptors, e.g., FcγRIIIA (CD16), FcγRIIA (CD32A) or CD64, or to an inhibitory Fc receptor, e.g., FcyRIIB (CD32B). In one aspect, a CH2 domain in a polypeptide will furthermore have substantial loss of binding to the first component of complement (C1q).

Optionally the CH2 domain further comprises substitutions into human IgG1 of IgG2 residues to further reduce binding to Fcγ receptors. It has been shown that substitutions at positions 233-236 (EU numbering) and IgG4 residues at positions 327, 330 and 331 (EU numbering) were shown to greatly reduce binding to Fcγ receptors and thus ADCC and CDC. Furthermore, Idusogie et al. (2000) J Immunol. 164(8):4178-84 demonstrated that alanine substitution at different positions, including K322, significantly reduced complement activation. In one aspect, a CH2 domain that retains binding to human FcRn but has reduction of binding to Fcγ receptors will lack or have modified N-linked glycosylation, e.g. at residue N297 (Kabat EU). In one aspect, a CH2 domain that loses binding to a Fcγ receptor will comprise at least one amino acid modification (for example, possessing 1, 2, 3, 4, 5, 6, 7, 8, 9, or more amino acid modifications) in the CH2 domain of the Fc region.

In one aspect the monomeric polypeptide will bind to FcRn, with a 1:1 ratio (1 FcRn for each monomeric polypeptide).

The monomeric Fc domain-containing polypeptides can retain partial FcRn binding (compared, e.g., to a wild type full length human IgG1 antibody). Optionally the monomeric polypeptide is capable of binding to human FcRn with intermediate affinity, e.g. retains binding to FcRn but has decreased binding to a human FcRn receptor compared to a full-length wild type human IgG1 antibody. The Fc moiety may further comprise one or more amino acid modifications, e.g. in the CH2 domain, that modulates binding to one or more Fcγ receptors.

In one aspect of any of the polypeptides herein, a tandem-CH3 domain containing polypeptide is present substantially in monomeric form (i.e. as a single polypeptide chain). This may be the case where the tandem-CH3 domain containing polypeptide a final product, or when the tandem-CH3 domain containing polypeptide is a production intermediate in the case where the polypeptide is a multimeric (e.g. dimeric) polypeptide.

### Uses of compounds

In one aspect, provided are the use of any of the compounds defined herein for the manufacture of a pharmaceutical preparation for the treatment or diagnosis of a mammal being in need thereof. Provided also are the use any of the compounds defined above as a medicament or an active component or active substance in a medicament. In a further aspect provided is a method for preparing a pharmaceutical composition containing a compound as defined above, to provide a solid or a liquid formulation for administration orally, topically, or by injection. Such a method or process at least comprises the step of mixing the compound with a pharmaceutically acceptable carrier.

In one aspect, provided is a method to treat, prevent or more generally affect a predefined condition by exerting a certain effect, or detect a certain condition using a compound herein, or a (pharmaceutical) composition comprising a compound disclosed herein.

The polypeptides described herein can be used to prevent or treat disorders that can be treated with antibodies, such as cancers, solid and non-solid tumors, hematological malignancies, infections such as viral infections, and inflammatory or autoimmune disorders.

In one aspect, the antigen of interest is expressed on the surface of a malignant cell of a type of cancer selected from the group consisting of: carcinoma, including that of the bladder, head and neck, breast, colon, kidney, liver, lung, ovary, prostate, pancreas, stomach, cervix, thyroid and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma and Burketts lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including neuroblastoma and glioma; tumors of the central and peripheral nervous system, including astrocytoma, neuroblastoma, glioma, and schwannomas; tumors of mesenchymal origin, including fibrosarcoma, rhabdomyoscaroma, and osteosarcoma; and other tumors, including melanoma, xeroderma pigmentosum, keratoacanthoma, seminoma, thyroid follicular cancer and teratocarcinoma, hematopoietic tumors of lymphoid lineage, for example T-cell and B-cell tumors.

In one aspect, polypeptides described herein can be used to prevent or treat a cancer selected from the group consisting of: carcinoma, including that of the bladder, head and neck, breast, colon, kidney, liver, lung, ovary, prostate, pancreas, stomach, cervix, thyroid and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma and Burketts lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including neuroblastoma and glioma; tumors of the central and peripheral nervous system, including astrocytoma, neuroblastoma, glioma, and schwannomas; tumors of mesenchymal origin, including fibrosarcoma, rhabdomyoscaroma, and osteosarcoma; and other tumors, including melanoma, xeroderma pigmentosum, keratoacanthoma, seminoma, thyroid follicular cancer and teratocarcinoma. Other exemplary disorders that can be treated according to the disclosure include hematopoietic tumors of lymphoid lineage, for example T-cell and B-cell tumors.

In one aspect, the methods of treatment comprise administering to an individual a multispecific polypeptide in a therapeutically effective amount. A therapeutically effective amount may be any amount that has a therapeutic effect in a patient having a disease or disorder (or promotes, enhances, and/or induces such an effect in at least a substantial proportion of patients with the disease or disorder and substantially similar characteristics as the patient).

The multispecific polypeptides can be included in kits. The kits may optionally further contain any number of polypeptides and/or other compounds, e.g., 1, 2, 3, 4, or any other number of multispecific polypeptide and/or other compounds. It will be appreciated that this description of the contents of the kits is not limiting in any way. For example, the kit may contain other types of therapeutic compounds. Optionally, the kits also include instructions for using the polypeptides, e.g., detailing the herein-described methods.

Also provided are pharmaceutical compositions comprising the compounds as defined above. A compound may be administered in purified form together with a pharmaceutical carrier as a pharmaceutical composition. The form depends on the intended mode of administration and therapeutic or diagnostic application. The pharmaceutical carrier can be any compatible, nontoxic substance suitable to deliver the compounds to the patient. Pharmaceutically acceptable carriers are well known in the art and include, for example, aqueous solutions such as (sterile) water or physiologically buffered saline or other solvents or vehicles such as glycols, glycerol, oils such as olive oil or injectable organic esters, alcohol, fats, waxes, and inert solids A pharmaceutically acceptable carrier may further contain physiologically acceptable compounds that act for example to stabilize or to increase the absorption of the compounds Such physiologically acceptable compounds include, for example, carbohydrates, such as glucose, sucrose or dextrans, antioxidants, such as ascorbic acid or glutathione, chelating agents, low molecular weight proteins or other stabilizers or excipients One skilled in the art would know that the choice of a pharmaceutically acceptable carrier, including a physiologically acceptable compound, depends, for example, on the route of administration of the composition Pharmaceutically acceptable adjuvants, buffering agents, dispersing agents, and the like, may also be incorporated into the pharmaceutical compositions.

The compounds can be administered parenterally. Preparations of the compounds for parenteral administration must be sterile. Sterilization is readily accomplished by filtration through sterile filtration membranes, optionally prior to or following lyophilization and reconstitution. The parenteral route for administration of compounds is in accord with known methods, e.g. injection or infusion by intravenous, intraperitoneal, intramuscular, intraarterial, or intralesional routes. The compounds may be administered continuously by infusion or by bolus injection. A typical composition for intravenous infusion could be made up to contain 100 to 500 ml of sterile 0.9% NaCl or 5% glucose optionally supplemented with a 20% albumin solution and 1 mg to 10 g of the compound, depending on the particular type of compound and its required dosing regimen. Methods for preparing parenterally administrable compositions are well known in the art.

### EXAMPLES

### Example 1: Construction of bispecific monomeric tandem CH3 Fc polypeptides Materials and Methods

Different constructs were made for use in the preparation of a bispecific Fc-polypeptide based on a scFv specific for human tumor antigen CD19 (anti-CD19 scFv) and a scFV specific for human activating receptor NKp46 on NK cells (anti-NKp46 scFv).

Shown below in Table 1 are the light chain and heavy chain variable regions amino acid sequences for the anti-CD19 component of the proteins.

**Table 1**

| **Anti-CD19** | **Sequence** |
|---|---|
| Anti-CD19-VK DNA | SEQ ID NO: 3 |
| Anti-CD19-VK amino acid | SEQ ID NO: 4 |
| Anti-CD19-VH DNA | SEQ ID NO: 5 |
| Anti-CD19-VH amino acid | SEQ ID NO: 6 |

Shown below in Table 2 are the light chain and heavy chain variable regions and scFv amino acid sequences for the anti-NKp46 component of the proteins.

**Table 2**

| **Anti-NKp46** | **scFV sequence (VHVK) / - stop** |
|---|---|
| NKp46-3 VH amino acid | SEQ ID NO: 7 |
| NKp46-3 VL amino acid | SEQ ID NO: 8 |
| NKp46-3 scFv amino acid | SEQ ID NO: 9 |

### Cloning and production of the recombinant proteins

Coding sequences were generated by direct synthesis and/or by PCR. PCR were performed using the PrimeSTAR MAX DNA polymerase (Takara, #R045A) and PCR products were purified from 1% agarose gel using the NucleoSpin gel and PCR clean-up kit (Macherey-Nagel, #740609.250). Once purified the PCR product were quantified prior to the In-Fusion ligation reaction performed as described in the manufacturer's protocol (ClonTech, #ST0345). The plasmids were obtained after a miniprep preparation run on an EVO200 (Tecan) using the Nucleospin 96 plasmid kit (Macherey-Nagel, #740625.4). Plasmids were then sequenced for sequences confirmation before to transfecting the CHO cell line.

CHO cells were grown in the CD-CHO medium (Invitrogen) complemented with phenol red and 6 mM GlutaMax. The day before the transfection, cells are counted and seeded at 175.000 cells/ml. For the transfection, cells (200.000 cells/transfection) are prepared as described in the AMAXA SF cell line kit (AMAXA, #V4XC-2032) and nucleofected using the DS137 protocol with the Nucleofector 4D device. All the tranfections were performed using 300 ng of verified plasmids. After transfection, cells are seeded into 24 well plates in prewarmed culture medium. After 24H, hygromycine B was added in the culture medium (200 µg/ml). Protein expression is monitored after one week in culture. Cells expressing the proteins are then sub-cloned to obtain the best producers. Sub-cloning was performed using 96 flat-bottom well plates in which the cells are seeded at one cell per well into 200 µl of culture medium complemented with 200 µg/ml of hygromycine B. Cells were left for three weeks before to test the clone's productivity.

Recombinant proteins which contain a IgG1-Fc fragment are purified using Protein-A beads (- rProteinA Sepharose fast flow, GE Healthcare, ref.: 17-1279-03). Briefly, cell culture supernatants were concentrated, clarified by centrifugation and injected onto Protein-A columns to capture the recombinant Fc containing proteins. Proteins were eluted at acidic pH (citric acid 0.1M pH3), immediately neutralized using TRIS-HCL pH8.5 and dialyzed against 1X PBS. Recombinant scFv which contain a "six his" tag were purified by affinity chromatography using Cobalt resin. Other recombinant scFv were purified by size exclusion chromatography (SEC).

### Format 3 (F3): CD19-F3-NKp46-3

The domain structure of F3 polypeptides is shown in Figure 1A. The DNA and amino acid sequences for the Fc portion comprised a tandem CH3 domain in which the two CH3 domains on the same polypeptide chain associated with one another, thereby preventing dimerization between different bispecific proteins.

The single chain polypeptide has domains arranged (N- to C- terminal) as follows:
(VK-VH)^{anti-CD19} - CH2 - CH3 - CH3 - (VH-VK)^{anti-NKp46}

The (VK-VH) units were made up of a VH domain, a linker and a VK unit (scFv).
Proteins were cloned, produced and purified as above. Bispecific protein was purified from cell culture supernatant by affinity chromatography using prot-A beads and analysed and purified by SEC. The protein showed a high production yield of 3.4 mg/L and with a simple SEC profile. The amino acid sequence for the F3 protein is shown in SEQ ID NO: 10.

### Format 4 (F4) : CD19-F4-NKp46-3

The protein having domain arrangment scFvCD19 - CH2 (N297S) -CH3 - CH3 - scFvNkp46-3 shown in Figure 1A as "Format 4" was constructed. The DNA and amino acid sequences for the Fc portion comprised a tandem CH3 domain as in Format F3, however additionally comprising a N297S mutation to prevent N-linked glycosylation and abolish FcγR binding. Proteins were cloned, produced and purified as above. Bispecific proteins was purified from cell culture supernatant by affinity chromatography using prot-A beads and analysed and purified by SEC. The protein showed a good production yield of 1mg/L and with a simple SEC profile. The amino acid sequence for the F4 protein with NKp46-3 variable domains is shown in SEQ ID NO: 11.

### Format 9 (F9) : CD19-F9-NKp46-3

The F9 polypeptide is a trimeric polypeptide having a central polypeptide chain and two polypeptide chains each of which associate with the central chain via CH1-CK dimerization. The domain structure of the trimeric F9 protein is shown in Figure 1B, wherein the bonds between the CH1 and CK domains are interchain disulfide bonds. The two antigen binding domains have a F(ab) structure permitting the use of antibodies irrespective of whether they remain functional in scFv format. The DNA and amino acid sequences for the Fc portion comprised a tandem CH3 domain as in Format F4 and a CH2 domain comprising a N297S substitution. Three variants of F9 proteins were produced: (a) cysteine residues in the hinge region left intact (wild-type, referred to as F9A), (b) cysteine residues in the hinge region replaced by serine residues (F9B), and (c) a linker sequence GGGSS replacing residues DKTHTCPPCP in the hinge (F9C). Variants F9B and F9C provided advantages in production by avoiding formation of homodimers of the central chain. The heterotrimer is made up of:
(1) a first (central) polypeptide chain having domains arranged as follows (N- to C-terminal):
   VH^{anti-CD19}-CH1 - CH2- CH3 - CH3 - VH^{anti-NKp46} - CK
   and
(2) a second polypeptide chain having domains arranged as follows (N- to C- terminal): VK^{anti-NKp46} - CH1
   and
(3) a third polypeptide chain having domains arranged as follows (N- to C- terminal): VK ^{anti-CD19} - CK

Proteins were cloned, produced and purified as above. Bispecific proteins was purified from cell culture supernatant by affinity chromatography using prot-A beads and analysed and purified by SEC. The protein showed a high production yield of 8.7 mg/L (F9A) and 3.0 mg/L (F9B), and with a simple SEC profile.

The amino acid sequences of the three chains of the F9 protein variant F9A are shown in the SEQ ID NOS: 12, 13 and 14. The amino acid sequences of the three chains of the F9 protein variant F9B are shown in the SEQ ID NOS: 12, 15 and 14. The amino acid sequences of the three chains of the F9 protein variant F9C are shown in the SEQ ID NOS: 12, 16 and 14.

### Format 10 (F10): CD19-F10-NKp46-3

The F10 polypeptide is a dimeric protein having a central polypeptide chain and a second polypeptide chain which associates with the central chain via CH1-CK dimerization. The domain structure of the dimeric F10 proteins is shown in Figure 1B wherein the bonds between the CH1 and CK domains are interchain disulfide bonds. One of the two antigen binding domains has a Fab structure, and the other is a scFv. The DNA and amino acid sequences for the Fc portion comprised a tandem CH3 domain as in Format F4 and a CH2 domain with a N297S substitution. Additionally, three variants of F10 proteins were produced: (a) cysteine residues in the hinge region left intact (wild-type, referred to as F10A), (b) cysteine residues in the hinge region replaced by serine residues (F10B, and (c) a linker sequence GGGSS (SEQ ID NO: 28) replacing residues DKTHTCPPCP (SEQ ID NO: 29) in the hinge (F10C). Variants F10B an F10C provided advantages in production by avoiding formation of homodimers of the central chain. The (VK-VH) unit was made up of a VH domain, a linker and a VK unit (scFv). The heterodimer is made up of:
(1) a first (central) polypeptide chain having domains arranged as follows (N- to C-terminal):
   VH^{anti-CD19} - CH1 - CH2- CH3 - CH3 - (VH - VK)^{anti-NKp46}
   and
(2) a second polypeptide chain having domains arranged as follows (N- to C- terminal): VK ^{anti-CD19} - CK.

Proteins were cloned, produced and purified as above. Bispecific proteins was purified from cell culture supernatant by affinity chromatography using prot-A beads and analysed and purified by SEC. The protein showed a good production yield of 2 mg/L (F10A) and with a simple SEC profile. The amino acid sequences of the two chains of the F10A protein variant are shown in the SEQ ID NO: 17 (second chain) and SEQ ID NO: 18 (first chain). The amino acid sequences of the two chains of the F10B protein variant are shown in the SEQ ID NO: 17 (second chain) and SEQ ID NO: 19 (first chain). The amino acid sequences of the two chains of the F10C protein variant are shown in the SEQ ID NO: 17 (second chain) and SEQ ID NO: 20 (first chain).

### Format 11 (F11): CD19-F11-NKp46-3

The domain structure of F11 polypeptides is shown in Figure 1C. The heterodimeric protein is similar to F10 but the structures of the antigen binding domains are reversed. One of the two antigen binding domains has a Fab-like structure, and the other is a scFv. The heterodimer is made up of
(1) a first (central) polypeptide chain having domains arranged as follows (N- to C-terminal):
   (VK - VH)^{anti-CD19} -CH2- CH3 - CH3 - VH^{anti-NKp46} _ CK
   and
(2) a second polypeptide chain having domains arranged as follows (N- to C- terminal): VK^{anti-NKp46} - CH1.

Proteins were cloned, produced and purified as above. Bispecific proteins was purified from cell culture supernatant by affinity chromatography using prot-A beads and analysed and purified by SEC. The protein showed a good production yield of 2 mg/L and with a simple SEC profile. The amino acid sequences of the two chains of the F11 protein are shown in SEQ ID NO: 21 (chain 1) and SEQ ID NO: 22 (chain 2).

### Format 12 (F12): CD19-F12-NKp46-3

The domain structure of the dimeric F12 polypeptides is shown in Figure 1C, wherein the bonds between the CH1 and CK domains are disulfide bonds. The heterodimeric protein is similar to F11 but the CH1 and CK domains within the F(ab) structure are inversed. The heterodimer is made up of:
(1) a first (central) polypeptide chain having domains arranged as follows (N- to C-terminal):
   (VK - VH)^{anti-CD19} -CH2- CH3 - CH3 - VH^{anti-NKp46}_ CH1
   and
(2) a second polypeptide chain having domains arranged as follows (N- to C- terminal): VK^{anti-NKp46} - CK.

Proteins were cloned, produced and purified as above. Bispecific proteins was purified from cell culture supernatant by affinity chromatography using prot-A beads and analysed and purified by SEC. The protein showed a good production yield of 2.8 mg/L and with a simple SEC profile. The DNA and amino acid sequences for the F12 protein are shown below. The amino acid sequences of the two chains of the F12 protein are shown in SEQ ID NO: 23 (chain 1) and SEQ ID NO: 24 (chain 2).

### Format 17 (F17): CD19-F17-NKp46-3

The domain structure of the trimeric F17 polypeptides is shown in Figure 1C, wherein the bonds between the CH1 and CK domains are disulfide bonds. The heterodimeric protein is similar to F9 but the VH and VK domains, and the CH1 and CK, domains within the C-terminal F(ab) structure are each respectively inversed with their partner. The heterotrimer is made up of:
(1) a first (central) polypeptide chain having domains arranged as follows (N- to C-terminal):
   VH^{anti-CD19} - CH1 - CH2- CH3 - CH3 - VK^{anti-NKp46} - CH1
   and
(2) a second polypeptide chain having domains arranged as follows (N- to C- terminal): VH^{anti-NKp46} - CK
   and
(3) a third polypeptide chain having domains arranged as follows (N- to C- terminal): VK^{anti-CD19} - CK

Additionally, three variants of F17 proteins were produced: (a) cysteine residues in the hinge region left intact (wild-type, referred to as F17A), (b) cysteine residues in the hinge region replaced by serine residues (F10B, and (c) a linker sequence GGGSS (SEQ ID NO: 28) replacing residues DKTHTCPPCP (SEQ ID NO: 29) in the hinge (F17C). Proteins were cloned, produced and purified as above. The amino acid sequences of the three chains of the F17B protein are shown in SEQ ID NOS: 25, 26 and 27.

### Example 2:

### NKp46 binding affinity by bispecific proteins by Surface Plasmon Resonance (SPR)

### Biacore T100 general procedure and reagents

SPR measurements were performed on a Biacore T100 apparatus (Biacore GE Healthcare) at 25°C. In all Biacore experiments HBS-EP+ (Biacore GE Healthcare) and NaOH 10mM served as running buffer and regeneration buffer respectively. Sensorgrams were analyzed with Biacore T100 Evaluation software. Protein-A was purchase from (GE Healthcare). Human NKp46 recombinant proteins were cloned, produced and purified at Innate Pharma.

### Immobilization of Protein-A

Protein-A proteins were immobilized covalently to carboxyl groups in the dextran layer on a Sensor Chip CM5. The chip surface was activated with EDC/NHS (N-ethyl-N'-(3-dimethylaminopropyl) carbodiimidehydrochloride and N-hydroxysuccinimide (Biacore GE Healthcare)). Protein-A was diluted to 10 µg/ml in coupling buffer (10 mM acetate, pH 5.6) and injected until the appropriate immobilization level was reached (i.e. 2000 RU). Deactivation of the remaining activated groups was performed using 100 mM ethanolamine pH 8 (Biacore GE Healthcare).

### Binding study

The bispecific proteins were tested as different formats F3, F4, F9, F10, F11 having the anti-NKp46 variable regions from the NKp46-3 antibody, and compared to the NKp46-3 antibody as a full-length human IgG1.

Bispecific proteins at 1 µg/mL were captured onto Protein-A chip and recombinant human NKp46 proteins were injected at 5 µg/mL over captured bispecific antibodies. For blank subtraction, cycles were performed again replacing NKp46 proteins with running buffer.

### Affinity study

Monovalent affinity study was done following a regular Capture-Kinetic protocol recommended by the manufacturer (Biacore GE Healthcare kinetic wizard). Seven serial dilutions of human NKp46 recombinant proteins, ranging from 6.25 to 400 nM were sequentially injected over the captured Bi-Specific antibodies and allowed to dissociate for 10 min before regeneration. The entire sensorgram sets were fitted using the 1:1 kinetic binding model.

### Results

SPR showed that all of the bispecific polypeptides of formats F3, F4, F9, F10 and F11 retained binding to NKp46. Monovalent affinities and kinetic association and dissociation rate constants are shown below in the table 3 below.

**Table 3**

| Bispecific mAb | ka (1/Ms) | kd (1/s) | KD (M) |
|---|---|---|---|
| CD19-F3-NKp46-3 | 3.905E+5 | 0.01117 | 28E-09 |
| CD19-F4-NKp46-3 | 3.678E+5 | 0.01100 | 30E-09 |
| CD19-F9A-NKp46-3 | 2.070E+5 | 0.00669 | 32E-09 |
| CD19-F10A-NKp46-3 | 2.607E+5 | 0.00754 | 29E-09 |
| CD19-F11A-NKp46-3 | 3.388E+5 | 0.01044 | 30E-09 |
| NKp46-3 IgG1 | 2.224E+5 | 0.00433 | 20E-09 |

### Example 3:

### NKp46 mechanism of action

NKp46 x CD19 bispecific proteins having an arrangement according to the F3 format described in Example 1 were compared to rituximab (anti-CD20 ADCC inducing antibody), and a human IgG1 isotype control antibody for functional ability to direct CD16-/NKp46+ NK cell lines to lyse CD19-positive tumor target cells.

Briefly, the cytolytic activity of the CD16-/NKp46+ human NK cell line KHYG-1 was assessed in a classical 4-h ⁵¹Cr-release assay in U-bottom 96 well plates. Daudi or B221 cells were labelled with ⁵¹Cr (50 µCi (1.85 MBq)/1 x 10⁶ cells), then mixed with KHYG-1 at an effector/target ratio equal to 50:1, in the presence of test antibodies at dilution range starting from 10⁻⁷ mol/L with 1/5 dilution (n=8 concentrations)

After brief centrifugation and 4 hours of incubation at 37°C, 50µL of supernatant were removed and transferred into a LumaPlate (Perkin Elmer Life Sciences, Boston, MA), and ⁵¹Cr release was measured with a TopCount NXT beta detector (PerkinElmer Life Sciences, Boston, MA). All experimental conditions were analyzed in triplicate, and the percentage of specific lysis was determined as follows: 100 x (mean cpm experimental release - mean cpm spontaneous release)/ (mean cpm total release - mean cpm spontaneous release). Percentage of total release is obtained by lysis of target cells with 2% Triton X100 (Sigma) and spontaneous release corresponds to target cells in medium (without effectors or Abs).

### Results

In the KHYG-1 hNKp46 NK experimental model, each NKp46 x CD19 bispecific protein induced specific lysis of Daudi or B221 cells by human KHYG-1 hNKp46 NK cell line, while rituximab and human IgG1 isotype control (IC) antibodies did not.

### Example 4:

### Binding of different bispecific formats to FcRn

Affinity of different antibody formats for human FcRn was studied by Surface Plasmon Resonance (SPR) by immobilizing recombinant FcRn proteins covalently to carboxyl groups in the dextran layer on a Sensor Chip CM5. A chimeric full length anti-CD19 antibody having human IgG1 constant regions and NKp46 x CD19 bispecific proteins having an arrangement according to the F3, F4, F9, F10, or F11 formats described in Example 1 were tested; for each analyte, the entire sensorgram was fitted using the steady state or 1:1 SCK binding model.

### Biacore T100 general procedure and reagents

SPR measurements were performed on a Biacore T100 apparatus (Biacore GE Healthcare) at 25°C. In all Biacore experiments Acetate Buffer (50 mM Acetate pH5.6, 150 mM NaCl, 0.1% surfactant p20) and HBS-EP+ (Biacore GE Healthcare) served as running buffer and regeneration buffer respectively. Sensorgrams were analyzed with Biacore T100 Evaluation software. Recombinant mouse FcRn was purchase from R&D Systems.

### Immobilization of FcRn

Recombinant FcRn proteins were immobilized covalently to carboxyl groups in the dextran layer on a Sensor Chip CM5. The chip surface was activated with EDC/NHS (N-ethyl-N'-(3-dimethylaminopropyl) carbodiimidehydrochloride and N-hydroxysuccinimide (Biacore GE Healthcare)). FcRn proteins were diluted to 10 µg/ml in coupling buffer (10 mM acetate, pH 5.6) and injected until the appropriate immobilization level was reached (i.e. 2500 RU). Deactivation of the remaining activated groups was performed using 100 mM ethanolamine pH 8 (Biacore GE Healthcare).

### Affinity study

Monovalent affinity study was done following the Single Cycle Kinetic (SCK) protocol. Five serial dilutions of soluble analytes (antibodies and bi-specific molecules) ranging from 41.5 to 660 nM were injected over the FcRn (without regeneration) and allowed to dissociate for 10 min before regeneration. For each analyte, the entire sensorgram was fitted using the 1:1 SCK binding model.

### Results

Results are shown in Table 4 below. The bispecific proteins with monomeric Fc domains (F3, F4, F9, F10, F11) displayed binding to FcRn. Human IgG1/K Anti-CD19 antibody having dimeric Fc domains displayed a KD (nM) of 7.8.

**Table 4**

| **Antibody/Bispecific** | **SPR method** | **KD nM** |
|---|---|---|
| CD19-F3- NKp46-3 | Steady State | 474.4 |
| CD19-F4- NKp46-3 | Steady State | 711.7 |
| CD19-F9A- NKp46-3 | Steady State | 858.5 |
| CD19-F10A- NKp46-3 | Steady State | 432.8 |
| CD19-F11- NKp46-3 | Steady State | 595.5 |

## Claims

1. A polypeptide chain capable of binding to human neonatal Fc receptor (FcRn), comprising a first and a second CH3 domain separated by a flexible linker, wherein the polypeptide chain has a domain arrangement, from N- to C- terminal, selected from:
(ABD) -- linker - CH2 - CH3 - linker - CH3;
(ABD₁) - linker - CH2 - CH3 - linker - CH3 - linker - (ABD₂); and
(ABD₁) - linker - CH1 - CH2 - CH3 - linker - CH3 - linker - (ABD₂),
wherein ABD, ABD₁ and ABD₂ are each an antigen binding domain,
wherein the CH2 is fused to the CH3 domain and the CH1, if present, is fused to a CH2 domain via a hinge domain,
and wherein the flexible linker is of sufficient length to permit the first and a second CH3 domain to associate with one another via non-covalent interactions, and wherein the flexible linker is a peptide linker having between 10 and 30 residues and comprises glycine and serine residues.

2. The polypeptide chain of claim 1, wherein the amino acid sequence separating the first and a second CH3 domains is a peptide linker having the formula (G₄S)ₓ wherein x is 2, 3, 4, 5 or 6.

3. A protein comprising a polypeptide chain of any one of the above claims, and one or more further polypeptide chains that associate therewith.

4. The polypeptide or protein of any one of the above claims, wherein the polypeptide substantially lacks binding via its Fc domain to one or more human Fcγ receptors selected from the group consisting of: CD16, CD32A, CD32B and CD64.

5. The polypeptide or protein of any of the above claims, wherein the CH2 domain comprises an amino acid substitution to decrease binding to a human Fcγ receptor.

6. The polypeptide or protein of any of the above claims, wherein each ABD comprises the hypervariable regions, optionally the heavy and light chain CDRs, of an antibody.

7. The polypeptide or protein of any of the above claims, wherein an ABD comprises an immunoglobulin heavy chain variable domain and a light chain variable domain.

8. The polypeptide or protein of any of the above claims, wherein one of the antigen binding domains binds to a cancer antigen.

9. The polypeptide or protein of any of the above claims, wherein one of the antigen binding domains binds to a cell surface receptor on an immune effector cell.

10. The polypeptide or protein of any of the above claims, wherein one of the antigen binding domains binds to a cell surface receptor on an immune effector cell and the other of the antigen binding domains binds to a cancer, viral or bacterial antigen.

11. The polypeptide or protein of any of the above claims, wherein the cell surface receptor on an immune effector cell is a member of the immunoglobulin superfamily.

12. A pharmaceutical composition comprising a polypeptide or protein of any one of the above claims, and a pharmaceutically acceptable carrier.

13. A polypeptide or protein of any of claims 1-11, for use in the treatment of a cancer, infectious disease or an inflammatory or autoimmune disease.

## Patentansprüche

1. Polypeptidkette, die an den humanen neonatalen Fc-Rezeptor (FcRn) binden kann, umfassend eine erste und eine zweite CH3-Domäne, die durch einen flexiblen Linker getrennt sind, wobei die Polypeptidkette eine Domänenanordnung vom N- zum C-Terminal aufweist, ausgewählt aus:
(ABD) - Linker - CH2 - CH3 - Linker - CH3;
(ABD₁) - Linker - CH2 - CH3 - Linker - CH3 - Linker - (ABD₂); und
(ABD₁) - Linker - CH1 - CH2 - CH3 - Linker - CH3 - Linker - (ABD₂),
wobei ABD, ABD₁ und ABD₂ jeweils eine Antigenbindungsdomäne sind, wobei CH2 an die CH3-Domäne fusioniert ist und CH1, falls vorhanden, über eine Gelenkdomäne an eine CH2-Domäne fusioniert ist,
und wobei der flexible Linker von ausreichender Länge ist, um zu ermöglichen, dass die erste und eine zweite CH3-Domäne miteinander über nicht-kovalente Wechselwirkungen assoziieren, und wobei der flexible Linker ein Peptidlinker mit zwischen 10 und 30 Resten ist und Glycin- und Serin-Reste umfasst.

2. Polypeptidkette nach Anspruch 1, wobei die Aminosäuresequenz, die die erste und eine zweite CH3-Domäne trennt, ein Peptidlinker mit der Formel (G₄S)ₓ ist, wobei x 2, 3, 4, 5 oder 6 ist.

3. Protein, umfassend eine Polypeptidkette nach einem der obigen Ansprüche, und eine oder mehrere weitere Polypeptidkette(n), die damit assoziieren.

4. Polypeptid oder Protein nach einem der vorstehenden Ansprüche, wobei dem Polypeptid im Wesentlichen die Bindung über seine Fc-Domäne an einen oder mehrere humane Fcy-Rezeptoren, ausgewählt aus der Gruppe bestehend aus: CD16, CD32A, CD32B und CD64, fehlt.

5. Polypeptid oder Protein nach einem der vorstehenden Ansprüche, wobei die CH2-Domäne eine Aminosäuresubstitution umfasst, um die Bindung an einen menschlichen Fcy-Rezeptor zu verringern.

6. Polypeptid oder Protein nach einem der vorstehenden Ansprüche, wobei jedes ABD die hypervariablen Regionen, gegebenenfalls die schweren und leichten Ketten CDRs, eines Antikörpers umfasst.

7. Polypeptid oder Protein nach einem der vorstehenden Ansprüche, wobei ein ABD eine variable Domäne der schweren Kette eines Immunglobulins und eine variable Domäne der leichten Kette umfasst.

8. Polypeptid oder Protein nach einem der vorstehenden Ansprüche, wobei eine der Antigen-Bindungsdomänen an ein Krebsantigen bindet.

9. Polypeptid oder Protein nach einem der vorstehenden Ansprüche, wobei eine der Antigen-Bindungsdomänen an einen Zelloberflächenrezeptor auf einer Immuneffektorzelle bindet.

10. Polypeptid oder Protein nach einem der vorstehenden Ansprüche, wobei eine der Antigenbindungsdomänen an einen Zelloberflächenrezeptor auf einer Immuneffektorzelle bindet und die andere der Antigenbindungsdomänen an ein Krebs-, virales oder bakterielles Antigen bindet.

11. Polypeptid oder Protein nach einem der vorstehenden Ansprüche, wobei der Zelloberflächenrezeptor auf einer Immuneffektorzelle ein Mitglied der Immunglobulin-Superfamilie ist.

12. Pharmazeutische Zusammensetzung, umfassend ein Polypeptid oder Protein nach einem der obigen Ansprüche und einen pharmazeutisch verträglichen Träger.

13. Polypeptid oder Protein nach einem der Ansprüche 1-11 zur Verwendung bei der Behandlung von Krebs, Infektionskrankheit oder einer entzündlichen oder Autoimmunkrankheit.

## Revendications

1. Chaîne polypeptidique capable de se lier au récepteur Fc néonatal humain (FcRn), comprenant des premier et deuxième domaines CH3 séparés par un lieur flexible, laquelle chaîne polypeptidique a un agencement de domaines, de l'extrémité N à l'extrémité C, choisi parmi :
(ABD) - lieur - CH2 - CH3 - lieur - CH3 ;
(ABD₁) - lieur - CH2 - CH3 - lieur - CH3 - lieur - (ABD₂) ; et
(ABD₁) - lieur - CH1 - CH2 - CH3 - lieur - CH3 - lieur - (ABD₂),
dans laquelle chacun parmi ABD, ABD₁ et ABD₂ est un domaine de liaison à un antigène,
dans laquelle le CH2 est fusionné au domaine CH3 et le CH1, s'il est présent, est fusionné à un domaine CH2 via un domaine de charnière,
et dans laquelle le lieur flexible a une longueur suffisante pour permettre au premier et à un deuxième domaines CH3 de s'associer l'un à l'autre via des interactions non covalentes, et dans laquelle le lieur flexible est un lieur peptidique ayant entre 10 à 30 résidus et comprend des résidus de glycine et de sérine.

2. Chaîne polypeptidique selon la revendication 1, dans laquelle la séquence d'acides aminés séparant le premier et un deuxième domaines CH3 est un lieur peptidique de formule (G₄S)ₓ dans laquelle x vaut 2, 3, 4, 5 ou 6.

3. Protéine comprenant une chaîne polypeptidique selon l'une quelconque des revendications précédentes, et une ou plusieurs chaînes polypeptidiques qui s'associent à celle-ci.

4. Polypeptide ou protéine selon l'une quelconque des revendications précédentes, dans lequel le polypeptide est pratiquement dépourvu de liaison via son domaine Fc à un ou plusieurs récepteurs Fcy humains choisis dans le groupe constitué par : CD16, CD32A, CD32B et CD64.

5. Polypeptide ou protéine selon l'une quelconque des revendications précédentes, dans lequel le domaine CH2 comprend un remplacement d'acide aminé pour diminuer la liaison à un récepteur Fcy humain.

6. Polypeptide ou protéine selon l'une quelconque des revendications précédentes, dans lequel chaque ABD comprend les régions hypervariables, éventuellement les CDR de chaînes lourde et légère, d'un anticorps.

7. Polypeptide ou protéine selon l'une quelconque des revendications précédentes, dans lequel un ABD comprend un domaine variable de chaîne lourde d'immunoglobuline et un domaine variable de chaîne légère.

8. Polypeptide ou protéine selon l'une quelconque des revendications précédentes, dans lequel l'un des domaines de liaison à un antigène se lie à un antigène cancéreux.

9. Polypeptide ou protéine selon l'une quelconque des revendications précédentes, dans lequel l'un des domaines de liaison à un antigène se lie à un récepteur de surface cellulaire sur une cellule effectrice immunitaire.

10. Polypeptide ou protéine selon l'une quelconque des revendications précédentes, dans lequel l'un des domaines de liaison à un antigène se lie à un récepteur de surface cellulaire sur une cellule effectrice immunitaire et l'autre des domaines de liaison à un antigène se lie à un antigène cancéreux, viral ou bactérien.

11. Polypeptide ou protéine selon l'une quelconque des revendications précédentes, dans lequel le récepteur de surface cellulaire sur une cellule effectrice immunitaire est un membre de la superfamille des immunoglobulines.

12. Composition pharmaceutique comprenant un polypeptide ou une protéine selon l'une quelconque des revendications précédentes, et un véhicule pharmaceutiquement acceptable.

13. Polypeptide ou protéine selon l'une quelconque des revendications 1 à 11, pour une utilisation dans le traitement d'un cancer, d'une maladie infectieuse ou d'une maladie inflammatoire ou auto-immune.
